# EUROPEAN PATENT APPLICATION

(11) **EP 3 622 990 A1**
(43) Date of publication of application: **18.03.2020**
(21) Application number: 18194962.9
(22) Date of filing: 17.09.2018
(51) Int. Cl.: A61M 15/00

(54) **DRY POWDER INHALER**

(71) Applicant: Vectura Delivery Devices Limited, Chippenham, Wiltshire SN14 6FH (GB)
(72) Inventor: CLARKE, Roger, Cambridge, Cambridgeshire CB4 0GZ (GB); MELINIOTIS, Andreas, Cambridge, Cambridgeshire CB4 0GZ (GB)
(74) Representative: Clarke, Christopher John

(57) **Abstract**

The present invention provides an inhaler comprising a housing which contains a blister strip having a plurality of blisters which contain powdered medicament for inhalation, a mouthpiece mounted to the housing through which the medicament is inhaled by a user, an indexing and opening mechanism for indexing the blister strip and for opening the blisters which is operated by an actuator, wherein the blister strip is indexed by forward motion of the actuator from a first position to a second position, such as by opening a cover, and is also indexed in the same direction by reverse motion of the actuator from the second positon to the first position, such as by closing the cover.

## Description

### Technical Field of the Invention

The present invention relates to a dry powder inhaler with a blister strip containing doses of one or more substances for inhalation.

### Background to the Invention

Inhalers provide an attractive method for administering medicaments, for example to treat local diseases of the airway or to deliver drugs to the bloodstream via the lungs. The medicament is commonly provided as a dry powder pre-packaged in individual doses, such as capsules or blisters. It is advantageous for the inhaler to hold a number of doses so that there is no need to insert a blister into the device each time it is used. Therefore, many inhalers include means for storing a number of doses, e.g. in the form of a blister strip. Such devices are disclosed in, for example, WO 05/037353, WO 12/069854 and WO13/175176.

In the treatment of respiratory disorders it is often beneficial to administer a combination of active pharmaceutical ingredients (APIs) to a patient, for example a bronchodilator and an anti-inflammatory drug, such as salmeterol and fluticasone, or a triple combination such as a long acting β2-agonist (LABA), a long-acting muscarinic antagonist (LAMA) and a corticosteroid. However, the APIs typically have very different physicochemical properties; this affects, for example, their interactions with carrier particles. Consequently, it is very difficult to co-formulate two or three APIs in a single powder with the desired aerosolization properties.

One way to circumvent this problem is separate the APIs. For example, WO 00/064519 discloses a bulk powder device with separate reservoirs for each API. On actuation of the device, a dose of each formulation is dispensed, which is aerosolized as the patient inhales. WO 05/014089 discloses a device which has two separate blister strips, each containing an independent drug blend. The strips are indexed and opened concurrently when the device is actuated, so that the patient receives both APIs on inhalation. However, these devices are necessarily more complex than devices which dispense a single formulation.

WO 09/092520 discloses inhalers which can deliver different inhalation formulations during each use. In an indexing operation, a mouthpiece cover is pivoted from a closed to an open position by the user. The first phase of the cover opening causes an actuator to withdraw the piercer from the previously opened and emptied blister pocket. The second phase of the cover opening causes an indexing wheel to move the blister strip onward to the next blister pocket. Once the cover has been fully opened, the user pivots the actuator back to the closed position, in order to pierce the newly aligned blister pocket. Finally, after inhalation, the user pivots the cover back to the closed position. The indexing wheel is disengaged from the cover in the closing step, so that the blister strip is not moved backwards. In one embodiment, the inhaler is adapted to move the blister strip onwards by two blister pockets in each indexing operation, and has two piercing elements for simultaneously piercing the blister pockets aligned with each piercing element. However, indexing forward by two blisters on opening the cover either requires that the angle through which the cover pivots is increased, or that the gear ratio between the cover and the indexing wheel is changed, so that the same pivot angle moves the blister strip twice as far. Typically the cover is pivoted through approximately 90° on opening / closing, which is sufficient to expose the mouthpiece. However, increasing this to approximately 180° would make the device less easy to use. Effectively only one edge of the device is always exposed, which severely restricts where the user can hold the main body of the device in one hand while opening the outer cover with the other. On the other hand, significantly increasing the torque required to pivot the cover also makes the inhaler harder to operate, especially for elderly patients. Hence, a need exists for improved devices which overcome these issues.

### Brief Description of the Invention

The present invention addresses these problems, and in a first aspect, it provides an inhaler comprising a housing which contains a blister strip having a plurality of blisters which contain powdered medicament for inhalation, a mouthpiece mounted to the housing through which the medicament is inhaled by a user, an indexing and opening mechanism for indexing the blister strip and for opening the blisters which is operated by an actuator, wherein the blister strip is indexed by forward motion of the actuator from a first position to a second position, characterized in that the blister strip is also indexed in the same direction by reverse motion of the actuator from the second positon to the first position.

The inhaler of the invention allows the contents of two or more blisters to be dispensed on each actuation, for example from a blister strip that contains two or more different formulations. When the user inhales through the mouthpiece, an airflow is generated through the opened blisters to entrain the medicament and carry it via the mouthpiece, into the user's airway. It also allows the effort that is required for indexing and piercing to be distributed over both the forward and reverse motions (such as opening and closing a cover), so that the torque that must be applied by the user to operate the device is reduced.

Preferably the indexing and opening mechanism is arranged to index the blister strip forwards by one blister on each of the forward and reverse motions of the actuator and to open two blisters. Thus, on inhalation, the user receives the contents of two blisters. More preferably the two blisters contain different medicaments.

Preferably the inhaler has an outer cover which is pivotally mounted on the housing.

In one embodiment, the outer cover forms the actuator so that motion of the outer cover causes indexing of the blister strip and opening of the blisters. Preferably in the first position the outer cover is closed so that the mouthpiece is covered, and in the second position the outer cover is open so that the mouthpiece is exposed.

In an alternative embodiment, the inhaler has a lever which forms the actuator so that motion of the lever causes indexing of the blister strip and opening of the blisters.

Preferably, the opening means comprises a piercer and the actuating mechanism indexes one or more blisters into alignment with the piercer for piercing. More preferably, the blister strip is indexed during a first part of the forward motion of the actuator, the piercer pierces one or more aligned blisters during a second part of the forward motion, the piercer is removed from the blister(s) during a first part of the reverse motion of the actuator and the blister strip is indexed again during a second part of the reverse motion.

In one embodiment, the indexing and opening mechanism comprises first and second drive gears, an idler gear and a blister strip indexing wheel, wherein the actuator is drivingly linked to the first drive gear, and to the second drive gear via the idler gear so that the first and second drive gears rotate in opposite senses during motion of the actuator; wherein the first drive gear drives the indexing wheel during at least part of the forward motion of the actuator, and does not drive the indexing wheel during the reverse motion of the actuator; and the second drive gear drives the indexing wheel during at least part of the reverse motion of the actuator, and does not drive the indexing wheel during the forward motion of the actuator. The term "drivingly linked" includes both direct engagement and indirect linkage that transmits drive, such as via one or more intermediate gears.

Preferably, the indexing and opening mechanism comprises first and second actuator gears which are connected to and driven by the actuator, wherein the first actuator gear drives the first drive gear, and the second actuator gear drives the idler gear which in turn drives the second drive gear.

Preferably, the first and second drive gears are axially linked together to form a shuttle whilst being free to rotate independently, wherein the first and / or second drive gear has a track follower which interacts with a track formation on the housing to cause the shuttle to translate axially relative to the indexing wheel so that the first and second drive gears engage and disengage with the indexing wheel. More preferably, the first and second drive gears each have a ramp follower which interacts with ramps on the housing to cause the first and second drive gears to disengage with the indexing wheel in the first and second positions, and preferably also during piercing and removal of the piercer.

Alternatively, a first drive coupling is connected to the first drive gear, a second drive coupling is connected to the second drive gear, and the drive couplings engage and disengage with the indexing wheel.

In another embodiment, the indexing and opening mechanism comprises first and second drive gears that are connected to a blister strip indexing wheel, and an idler gear, wherein the actuator is drivingly linked to the first drive gear during at least part of the forward motion of the actuator, and is not drivingly linked to the first drive gear during the reverse motion of the actuator; and the actuator is drivingly linked to the idler gear and the second drive gear during at least part of the reverse motion of the actuator, and is not drivingly linked to the second drive gear during the reverse motion of the actuator. Preferably, a dual drive coupling is connected to and driven by the actuator, and the dual drive coupling goes into and out of driving linkage with the first and second drive gears.

Preferably, the indexing and opening mechanism comprises a first actuator gear which drives the first drive gear and a second actuator gear which drives the idler gear which in turn drives the second drive gear so that the first and second actuator gears rotate in opposite senses during motion of the actuator, and the dual drive coupling engages and disengages with the first and second actuator gears.

In a second aspect, the present invention provides an inhaler comprising a housing which contains a blister strip having a plurality of blisters which contain powdered medicament for inhalation, a mouthpiece mounted to the housing through which the medicament is inhaled by a user, an outer cover which is mounted on the housing so that it pivots between a first, closed position in which the outer cover covers the mouthpiece, and a second, open position in which the mouthpiece is exposed, and an indexing and piercing mechanism; wherein pivoting the outer cover from the closed position to the open position causes the blister strip to be indexed by one blister and causes two blisters to be pierced, and wherein pivoting the outer cover from the open position to the closed position also causes the blister strip to be indexed by one blister in the same direction.

### Brief Description of the Figures

The invention will now be further described with reference to the Figures, wherein:
Figures 1A and 1B show an inhaler of the invention with the outer cover in the closed and opened positions respectively. Figure 1C shows the inhaler with the outer cover removed so that the internal components are visible.
Figure 2 shows the sequence of steps during operation of an inhaler according to the invention.
Figures 3A and 3B show expanded and assembled views of the actuating mechanism of a first embodiment of an inhaler according to the invention.
Figure 4A and 4B show perspective and side views of the drive coupling. Figure 4C shows the track formation
Figures 5A and 5B show expanded and assembled views of the actuating mechanism of the second embodiment.
Figure 6 shows the drive coupling
Figures 7A and 7B show the drive elements on the first and second actuator gears.
Figures 8A and 8B show expanded and assembled views of the actuating mechanism of the third embodiment.
Figure 9 shows the drive coupling
Figure 10 shows the drive pin which fits in a radial slot in the outer cover.
Figures 11A, 11B and 11C show the drive dogs on the gear selector and the actuator gear dogs in the first and second drive positions.
Figure 12 shows the track formation and the drive coupling in the different positions during one full opening and closing cycle.
Figures 13A and 13B show expanded and assembled views of the actuating mechanism of the fourth embodiment.
Figures 14A, 14B, 14C and 14D show perspective views from opposite sides of the first and second drive gears. Figure 14E shows a perspective view of the first and second drive gears connected together.
Figures 15A and 15B are perspective views of each side of the housing, showing the track formation and ramps.
Figures 16A and 16B schematically shows the positions of the drive gears and the indexing wheel (top row), the track follower and the track formation (middle row) and the ramps and ramp followers (bottom row) at several stages during opening and closing of the outer cover.

### Detailed Description of the invention

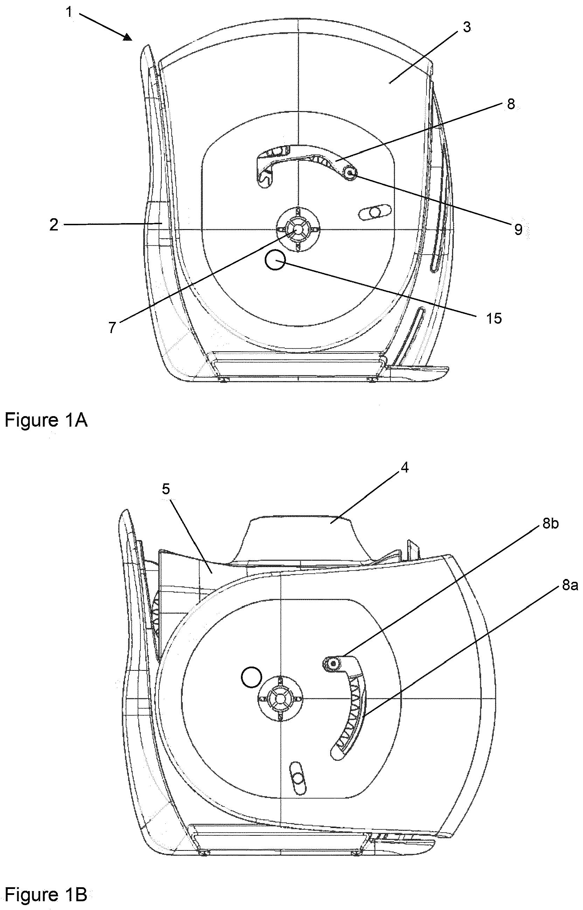

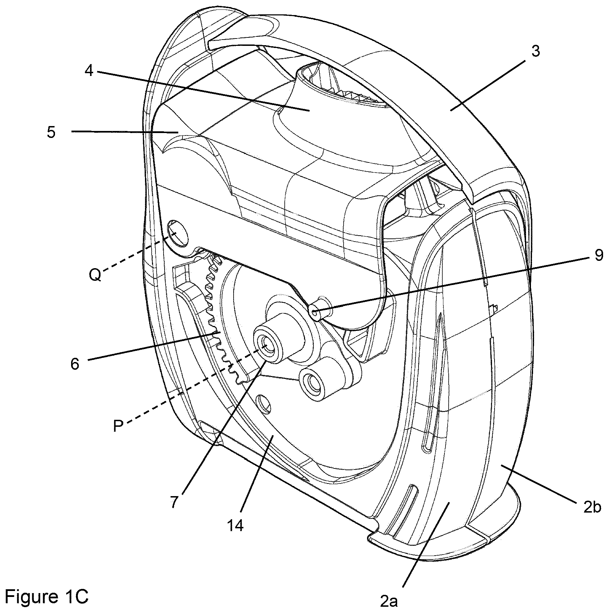

Figure 1 shows an inhaler according to the invention. The inhaler **1** has a housing **2** formed from two shell portions **2a, 2b** and an outer cover (or cap) **3.** The outer cover is pivotally mounted to the housing. The outer cover **3** can be rotated through approximately 90° from a closed position as shown in Figure 1A in which the outer cover **3** covers and protects a mouthpiece **4** to a fully open position, shown in Figure 1B in which the mouthpiece **4** is exposed to enable a user to inhale a dose of medicament.

Figure 1C shows the inhaler with the outer cover **3** partially cut away so that some of the internal components can be seen. The outer cover is mounted for rotation on axles **7** located on axis **P** on either side of the housing. The mouthpiece **4** is attached to or formed as part of a mouthpiece support member **5** which is pivotally mounted to the housing about a second axis **Q.** A piercer (not visible in Figure 1) is located on the underside of the support member **5** directly beneath the mouthpiece **4.** A cam **9** is located on one side of the mouthpiece support member **5** and cooperates with a cam slot **8** in the outer cover **3** to cause the mouthpiece support component and the piercer to pivot in a manner which is described below. Alternatively, the mouthpiece support component could be fixed to the housing, and the piercer may be pivotally mounted to the mouthpiece support component so that the piercer pivots relative to the mouthpiece.

The inhaler has a gear mechanism **6** (only part of which is visible) that selectively couples the outer cover to a blister strip indexing wheel. The outer cover is connected to the gear mechanism in a manner which is explained below. Pivoting the outer cover between the closed and open positions causes the indexing wheel to index (i.e. sequentially move) the blister strip and also causes the piercer to pierce one or more blisters. The user then inhales through the mouthpiece, which aerosolizes the powder in the pierced blister(s).

The inhaler of the invention differs from previous inhalers (such as those described in WO13/175177) in that the blister strip is moved forwards by both opening and closing the outer cover. This makes it possible, for example, to index and pierce a pair of blisters on each complete actuation (i.e. a full opening and closing cycle) of the device, so that the user inhales the contents of both blisters simultaneously.

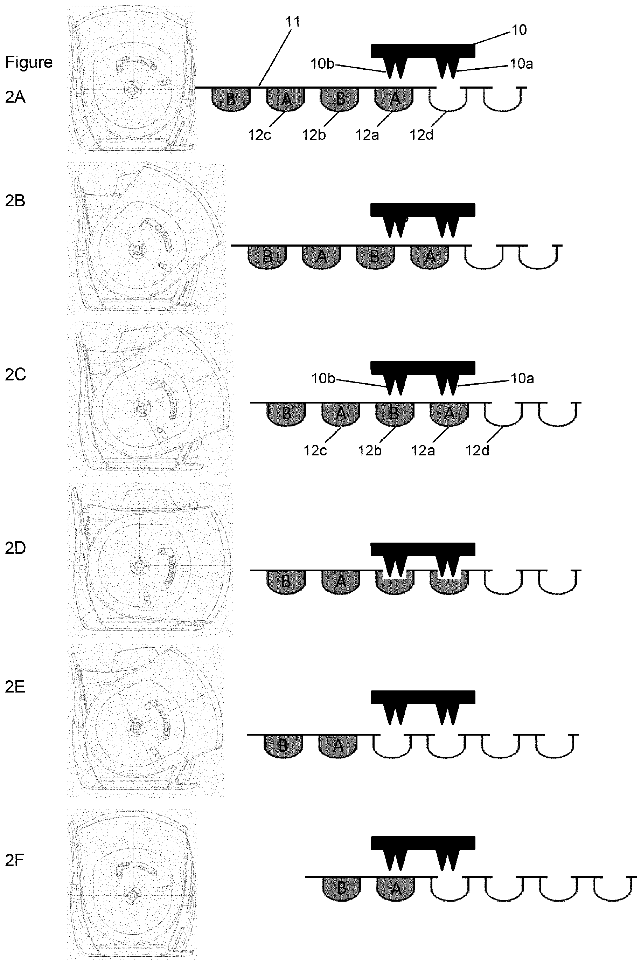

Figure 2 schematically shows a blister strip **11** suitable for use in the inhaler of the invention, having blisters **12** containing two different formulations. The blister strip is typically cold formed from a ductile foil laminate or a plastics material and includes a pierceable lid, typically foil or a foil laminate, which is heat-sealed around the periphery of the blister after the dose of medicament has been introduced during manufacture.

The blisters **12** are arranged in pairs, in which one blister of each pair contains a first formulation (A) and the other contains a second formulation (B) in alternating sequence (ABAB). Typically the two formulations contain different APIs, although the formulations could also contain, for example, a single API with two different particle sizes and / or mixed with different excipients and / or prepared by different processes.

The inhaler is configured to operate with this blister strip so that it indexes and opens two blisters on each complete actuation in a manner which will be explained below, so that the user inhales the contents of both blisters simultaneously.

As shown schematically in Figure 2, the piercer **10** has two piercing elements **10a, 10b** which face the blister strip **11,** one piercing element for each of the two blisters that are pierced in a single actuation. The piercing elements may be identical, but may also be different, for example if it is desired to create openings of different sizes in the two blisters, due to the different properties of the two formulations.

Each piercing element has a pair of piercing teeth, for creating two openings in the lid of each blister so that air is drawn into the blister through one opening and flows out of the blister together with an entrained dose of medicament through the other opening and via the mouthpiece into the user's airway. Alternatively, the piercing element may have a larger number of teeth for creating two or more inlet openings and/or two or more outlet openings; or the piercing element could create a single large opening which allows air to flow both into and out of the blister. The piercer is preferably formed as a single component.

The ABAB blister configuration has the advantage that each piercing element, and the associated airway to the mouthpiece, always interacts with the same formulation (i.e. either A or B, but never A in one actuation and then B in the next). Therefore the piercer and airway can be designed or customised to be optimal for that particular formulation.

Alternatively, an ABBA arrangement is possible, with an AB pair being followed on the next use by a BA pair. In each case, the user receives one blister of formulation A and one blister of formulation B (provided of course that the blister strip is correctly positioned initially). This configuration has the advantage that it may be simpler to manufacture, because the number of pairs of adjacent blisters which contain different formulations is halved.

The blister strip preferably has numbers printed on it that are visible to the user, for example through a window in the housing, in order to display the number of remaining (or used) doses. This is preferable to having a separate dose counter, because there is no possibility of the incorrect dose number being displayed. In a device which has two blister strips, either or both of the strips could be numbered. However, if one of the strips is not indexed correctly for any reason, then dose count could be incorrect or misleading. If only one strip is numbered, then the displayed number would be incorrect for one of the formulations, but the user would have no way of knowing this. If both strips are numbered then the displayed numbers would differ and the user would be unable to tell which is correct. Thus, an advantage of an advantage of having both formulations in a single blister strip is that the displayed number is always correct.

In a further configuration, each blister could contain the same formulation, so that the inhaler provides a double dose. Another possibility is for each blister to contain a half dose, so that opening two blisters provides a single dose. This has the advantage that variations in the amount of powder dispensed into each blister result in smaller variations in the total amount of powder dispensed on actuation. This is because, on average, too large an amount in one blister may be compensated by too small an amount in the other, leading to a reduction in the variability of the total amount of powder dispensed.

Although the inhaler of the invention is particularly suitable for indexing and piercing two blisters in each actuation, it can also be arranged to index and piece other numbers of blisters. For example, the inhaler may have a single piercing element and is correspondingly arranged to index the blister strip by one blister pitch on each complete actuation, i.e. to move the strip by half of the blister pitch on each of the opening and closing strokes. The inhaler can therefore operate in a conventional manner (one blister per operation). However, the angular motion of the outer cover that is available for indexing is doubled. The torque required to index the blister is reduced because the work is spread over two operations (opening and closing). The inhaler is therefore suitable for the elderly, infirm or other users who may find it difficult to operate a conventional inhaler, such as young children. Alternatively, the additional angular motion could be used for other purposes. For example, the force applied during piercing could be increased, so that, for example, blisters with tougher and / or thicker lids could be used, or larger openings could be created, or the piercer could pierce through both the lid and the base of each blister. Other possibilities include increasing the amount of force for crushing the used blisters, which would reduce the size of the used blister strip; or compressing a spring which, when released, could apply an impulse to the next unused blister and thereby help to de-agglomerate the powder.

Similarly, the inhaler may have three piercing elements and the gear mechanism may be correspondingly arranged to move the blister strip by one and a half blister pitches on each of opening and closing. Thus the inhaler can deliver three different formulations in a single operation from a blister strip having an ABCABC pattern, or a double dose of one formulation and a single dose of another from a blister strip having an AABAAB pattern. Likewise, it could deliver a triple dose of a single formulation. Furthermore, it is possible to use a blister strip containing four different formulations by indexing two blisters on each of opening and closing, and piercing four blisters. Whilst this could in principle also be achieved in an analogous manner to WO 09/092520 (i.e. indexing and piercing three or four blisters in a single actuating stroke), the torque required to do so would be very large, which could make the inhaler impractical. In contrast, spreading the work across the opening and closing strokes reduces the required torque to a manageable level. Other numbers and combinations of blisters are also possible, and those skilled in the art will be able to adapt the blister strip and inhaler accordingly.

The sequence of steps during indexing and piercing is schematically shown in Figure 2. Only six blisters are shown for illustration, but typically the strip actually has 30 or 60 blisters.

Indexing takes place during the first part of the opening movement (e.g. as the outer cover is pivoted from 0 to 60°). Piercing occurs during the remainder of the opening movement (e.g. as the outer cover is pivoted from 60 to 90°) while the blister strip is stationary. The blister strip is indexed from left to right. The first two unused blisters **12a, 12b** are the next ones to be pierced. To the left of these are further unused blisters **12c,** and to the right are used blisters **12d** which have already been pierced and whose contents have been inhaled. Figure 2A shows the configuration when the outer cover is closed. The leading unused blister **12a** is aligned with piercing element **10b.** The second unused blister has not yet been brought into alignment with the piercer. A used blister **12d** is aligned with the other piercing element **10a.**

As the user begins to rotate the outer cover, the blister strip indexed forwards. Figure 2B shows outer cover part way through the first part of the opening movement, with the blister strip having moved forwards by about two thirds of the blister pitch.

Figure 2C shows the configuration at the end of the first part of the opening movement. The blister strip has indexed forward by one blister, so that the first two unused blisters **12a, 12b** are aligned with the two piercing elements **10a, 10b.** Up to this point, the actuation of the inhaler is reversible, because piercing has not yet occurred. The user can abort by simply closing the outer cover, which moves the blister strip back to the position of Figure 2A.

When the outer cover is rotated beyond this point through the second part of the opening stroke, the indexing mechanism is disengaged, and the piercer begins to pivot so that the piercing elements **10a, 10b** pierce the two aligned and now stationary blisters **12a, 12b.** Figure 2D shows the fully open position, in which the blisters have been pierced. The user then inhales through the mouthpiece, which aerosolizes the powder in the pierced blister.

As the outer cover is closed, the piercing elements are withdrawn from the pierced blisters in the first part of the closing stroke (e.g. as the outer cover is pivoted from 90 to 60°) while the blister strip remains stationary, shown in Figure 2E.

Up to this point, the operation of the inhaler is similar to that of WO 13/175177, except that it has two piercing elements instead of one. However, in the inhaler of WO 13/175177, the blister strip remains stationary during the second part of the closing stroke. In contrast, the inhaler of the invention also indexes the blister strip during the second part of the closing stroke. This is achieved by using a different gear mechanism. Thus, as shown in Figure 2F, at the end of the closing stroke, the blister strip has been indexed forwards by a further blister. Consequently, the inhaler has indexed and pierced two blisters in one complete actuation. In Figure 2F, the inhaler is in the same state as in Figure 2A, except that two further blisters have been emptied.

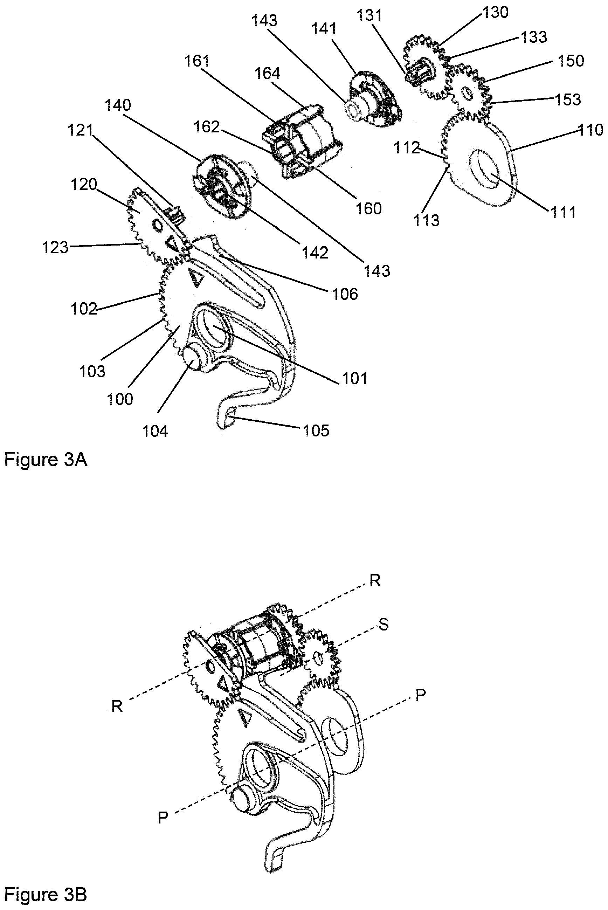

Figures 3A and 3B show expanded and assembled views of the actuating mechanism of a first embodiment of an inhaler according to the invention. The left hand side of mechanism is similar to that of WO 13/175177, comprising a first (opening) actuator gear **100,** a first drive gear **120,** a first drive coupling **140** and a blister strip indexing wheel **160.** However, unlike WO 13/175177, the mechanism additionally comprises a second (closing) drive coupling **141,** a second drive gear **130** and an idler gear wheel **150** which connects the second drive gear to a second actuator gear **110.**

The first and second actuator gears **100, 110** are formed as plate-like portions, each having a central pivot hole **101, 111** which fits onto the axles **7** (see Figure 1C) on either side of the housing, so that the actuator gears are mounted for rotation about the same axis **P** as the outer cover. The actuator gears **100, 110** each have gear elements **102, 112** which consist of teeth **103, 113** extending around about a quarter of the periphery.

The first and second actuator gears **100, 110** may be keyed or otherwise attached to the outer cover when the inhaler is assembled so that the first and second actuator gears and the outer cover rotate together. For example, a round post **104** protrudes from each actuator gear and is received in a corresponding hole **15** in the outer cover **3** (shown in Figure 1A). Opening or closing the outer cover thereby causes the actuator gears **100, 110** to rotate about axis **P.** Alternatively, the first and second actuator gears **100, 110** and outer cover may be formed as a single component, for example they may be moulded together as a unitary piece.

The first and second drive gears **120, 130** are mounted for rotation about a third axis **R** by means of shafts **121, 131.**

The first actuator gear element **102** transmits drive from the outer cover as it is rotated to the first drive gear **120** by means of the gear teeth **103** which mesh with corresponding gear teeth **123** on the first drive gear **120.**

The second actuator gear element **112** transmits drive from the outer cover as it is rotated via the idler gear wheel **150** to the second drive gear **130.** The idler gear wheel is mounted for rotation about a fourth axis, **S** on an idler gear axle on the housing. The gear teeth **113** of the actuator gear element mesh with the gear teeth **153** on the idler gear wheel which in turn mesh with the gear teeth **133** on the second drive gear **130.**

Thus the first drive gear **120** rotates in response to rotation of the first actuator gear **100,** and the second drive gear **130** rotates in response to rotation of the second actuator gear **110,** but in the opposite sense, due to the presence of the idler gear wheel **150.**

Each drive gear **120, 130** is fixedly attached to its respective drive coupling **140, 141** by its shaft **121, 131.** The shafts are cruciform in cross-section and fit into corresponding recesses **142** in the shafts **143** of the drive couplings (only the recess in the first drive coupling is visible in Figure 3A). Thus the drive couplings **140, 141** rotate together with their respective drive gear **120, 130** in response to rotation of the actuator gears **100, 110** at all times.

The indexing wheel **160** comprises a number of spokes **161** (typically four) extending from a hub **162.** The spokes are arranged so that a blister locates between the protruding ends **164** of successive spokes as the blister strip passes around the indexing wheel.

The manner in which the drive couplings **140, 141** selectively connect to and disconnect from the indexing wheel is described in detail in WO13/175177. Briefly, the drive couplings operate as follows. The shaft **143** of each drive coupling fits into a recess inside the hub **162** of the indexing wheel and provide bearing surfaces on which the indexing wheel **160** rotates. The indexing wheel is therefore coaxial with the drive gears **120, 130** on axis **R.** The drive couplings **140, 141** selectively connect and disconnect the drive gears **120, 130** to the indexing wheel **160,** so that, when coupled, the indexing wheel **160** rotates in response to rotation of the outer cover to index the blister strip.

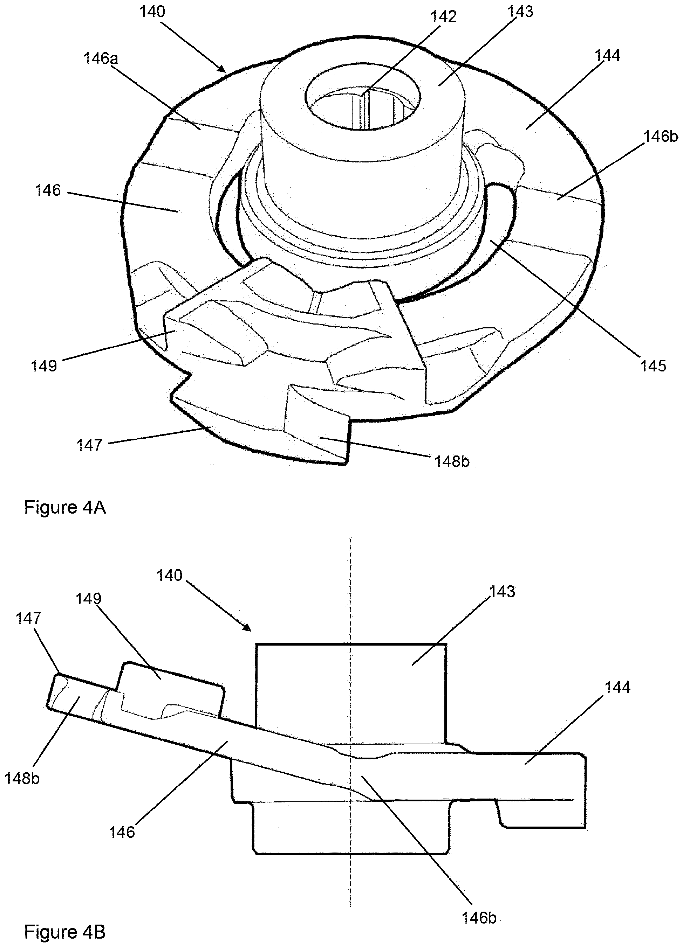

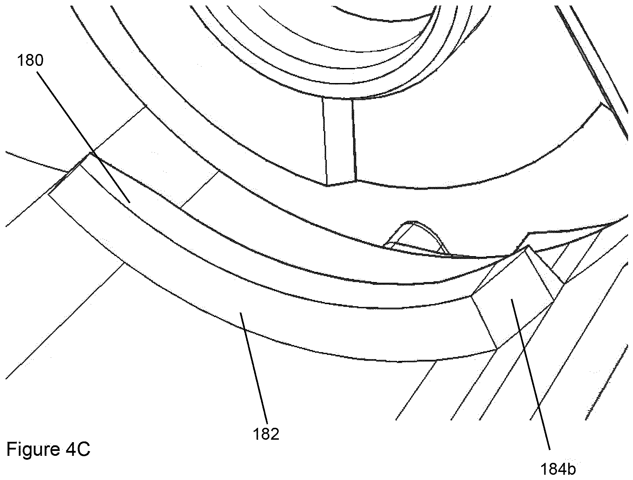

Figure 4A and 4B show perspective and side views of a drive coupling **140, 141.** Each drive coupling has a circular flange **144** that extends radially from one end of the shaft **143.** The flange **144** has a cut-away arcuate opening **145** where the flange **144** joins the shaft **143** so that approximately half **146** of the flange is not directly attached to the shaft **143** but only to the remaining portion of the flange **144** at each of its ends **146a, 146b.** As a result, this portion **146** of the flange **144** is flexible and can be deflected out of the plane of the flange **144** towards or away from the indexing wheel when force is applied to it. The flange **144** is made from a resilient material so that when the deflected flexible flange portion **146** is released, it returns to its neutral position, in which it is coplanar with the remaining fixed portion of the flange **144.** The flexible flange portion **146** has an integrally formed flange deflecting dog **147** projecting radially from its outer edge. The flange deflecting dog **147** has first and second angled engaging faces **148a, 148b** on opposite sides, only one of which is visible in Figure 4.

On the inside of each shell portion **2a, 2b** of the housing **2** there is a track formation **180,** one for each of the drive couplings. As shown in Figure 4C, the track formation **180** is in the form of a barrier which separates and defines tracks **182** on either side. Each end of the barrier has an angled face **184a,b** (only the track and angled engaing face on the outer side are visible in Figure 4C).

When the drive gear is rotated in a first direction, the first drive coupling **140** rotates together with it and the first angled engaging face **148a** on the flange deflecting dog **147** contacts the angled face **184a** of the barrier **180.** Further rotation of the drive coupling **140** causes the flange deflecting dog **147** to ride along the angled engaging face **184a** and onto the inner track **182,** thereby deflecting the flexible flange portion **146** towards the indexing wheel.

The flexible flange portion **146** has a wedge-shaped drive dog **149** that upstands inwardly towards the indexing wheel. The drive dog **149** engages between two spokes **161** of the indexing wheel **160** when the flexible flange portion **146** has been deflected inwardly, so that the indexing wheel **160** is now driven by the drive coupling **140.**

At the point when the flange deflecting dog **147** reaches the end of the inner track **182,** it falls off the barrier **180.** The resilience of the flexible flange portion **146** causes it to return to its neutral position, so that the drive dog **149** is no longer engaged between the spokes **161** of the indexing wheel **160.** This is the end of the first part of the opening stroke of the outer cover. The circumferential length of the barrier **180** controls the extent of rotation of the indexing wheel **160** relative to the extent of rotation of the drive gear **120.** In an embodiment where two blisters are indexed and pierced on each actuation, the length of the barrier is chosen so that the indexing wheel is rotated through the correct angle to move the next, unused blister, into alignment with the blister piercing element during the opening stroke.

Thus when the drive coupling is rotated further during the second part of the opening stroke, drive to the indexing wheel is disengaged. Despite continued rotation of the outer cover and drive gear in the same direction, the indexing wheel is stationary. This allows the blisters to be pierced while the blister strip is stationary, in a manner which is explained below.

As the outer cover is returned to the closed position, the first drive gear and first drive coupling rotate back in the opposite direction. The second angled surface **148b** of the flange deflecting dog **147** contacts the second angled face of the barrier **184b,** so that the flange deflecting dog **147** rides onto the other surface of the barrier **180** and travels along the outer track **182,** thereby causing the flexible flange portion **146** to deflect outwardly away from the indexing wheel **160.** This enables the drive coupling **140** to rotate in the opposite direction without any drive from the first drive gear **120** to the indexing wheel **160.**

So far, the mechanism is the same as that of WO 13/175177. However, in WO 13/175177 the blister strip is stationary while the outer cover is closed, whereas in this embodiment of the invention, the second actuator gear **110,** idler gear wheel **150,** second drive gear **130** and second drive coupling **141** also cause the blister strip also to be indexed as the outer cover is closed.

The second drive coupling **141** interacts with the second track formation in essentially the same manner as the first drive coupling and track formation, except that the second drive coupling **141** is disengaged from the indexing wheel **160** during the opening stroke and engages with the indexing wheel **160** during the second part of the closing stroke.

As the user closes the outer cover from the fully open position, in the first part of the closing movement, the piercing elements are removed from the blisters. The second actuator gear segment **112** drives the idler gear **150,** which in turn drives the second drive gear **130** and the second drive coupling **141.** However, because the second drive coupling **141** is not engaged with the indexing wheel **160,** the blister strip remains stationery. At the start of the second part of the closing stroke, second drive coupling **141** engages the indexing wheel **160** in the same manner as the first drive coupling **140** did during the first part of the opening stroke. Thus further closing the outer cover causes the indexing wheel to drive the blister strip. The idler gear **150** reverses the relative sense of rotation of the second drive gear **130** the second actuator gear **110.** The indexing wheel **160** is therefore rotated in the same sense as during the opening stroke, even though the outer cover and the second actuator gear rotate in the opposite sense. Thus the blister strip is indexed forwards, not backwards, by the closing of the cover.

Just before the outer cover reaches the fully closed position, the second drive coupling **141** disengages from the indexing wheel **160** in the same manner as the first drive coupling **140** did at the end of the first part of the forward stroke.

Thus the two drive couplings combine to change the gearing between the outer cover and the indexing wheel from forward during the first part of the opening stroke, into neutral during the second part of the opening stroke and the first part of the closing stroke, then into reverse during the second part of closing and finally into neutral again in the fully closed position. Consequently, the blister strip is indexed both during opening and closing. For example, the strip may be indexed by one blister on opening and by a second on closing, so that the next two unopened blisters are correctly positioned relative to the piercing elements, ready for the next use.

The outer cover cooperates with the piercer so that rotation of the outer cover during this second part of the opening stroke stage causes the piercer to pivot relative to the housing to puncture the lid of the aligned blisters, in essentially the same manner as also described in detail in WO 13/175177. In brief, this is achieved by means of a cam **9** located on the mouthpiece support member **5** which cooperates with the cam slot **8** in the outer cover **3.** As shown in Figure 1B, the cam slot has an arcuate portion **8a** centered on axis **P** and a leg portion **8b.** During rotation of the outer cover **3** through the first part of the opening stroke, the cam **9** slides along the arcuate portion **8a** of the cam slot without causing any movement of the mouthpiece support member **5** because the arcuate portion has the same axis as the outer cover. However, during the second part of the rotation of the outer cover, the cam **9** reaches the leg portion **8b** and engages the side walls of the cam slot **9** so as to cause the mouthpiece support member **5** to pivot about axis **Q,** thereby pulling the piercing elements into the lids of the aligned blisters and piercing them. When the outer cover is rotated back through the first part of the closing stroke, the cam **9** travels back along the leg portion **8b** of the cam slot, causing the mouthpiece support member **5** to pivot back to its initial position, thereby lifting the piercing elements out of the pierced blisters. During rotation of the outer cover through the second part of the closing stroke, the cam slides along the arcuate portion **8a** of the cam slot without causing any movement of the mouthpiece support member **5.** In an alternative configuration, the cam could project from the inside of the outer cover into a cam slot on the mouthpiece support member.

The first actuator gear may include a cantilever **105** which contacts a ramp **14** (shown in Figure 1C) on the housing **2** so that a biasing force is applied by the ramp **14** to the cantilever **105** during rotation of the first actuator gear **100,** as described in WO 13/175176. The shape of the ramp is designed so that the degree of deflection of the cantilever, and hence the biasing force changes with the angle of rotation of the actuator gear. As the degree of deflection of the cantilever changes, the torque that must be applied to the outer cover changes, thereby producing a tactile effect experienced by the user. The first actuator gear may also include a detent arm **106,** which has an enlarged head that engages with a detent peg on the housing (not visible in Figure 1C) when the outer cover is in the closed position. When the user begins to open the outer cover, a small torque must initially be applied to deflect the detent arm as the head passes over the detent peg. In addition to providing a slight resistance to initial movement of the outer cover which prevents accidental opening, the enlarged head is shaped so that the detent mechanism also pulls the cover in to the closed position at the end of the closing stroke. The detent arm can be arranged to generate a 'click' at end of the second part of the closing stroke, and so provide an audible and palpable signal to the user that the outer cover has been fully closed.

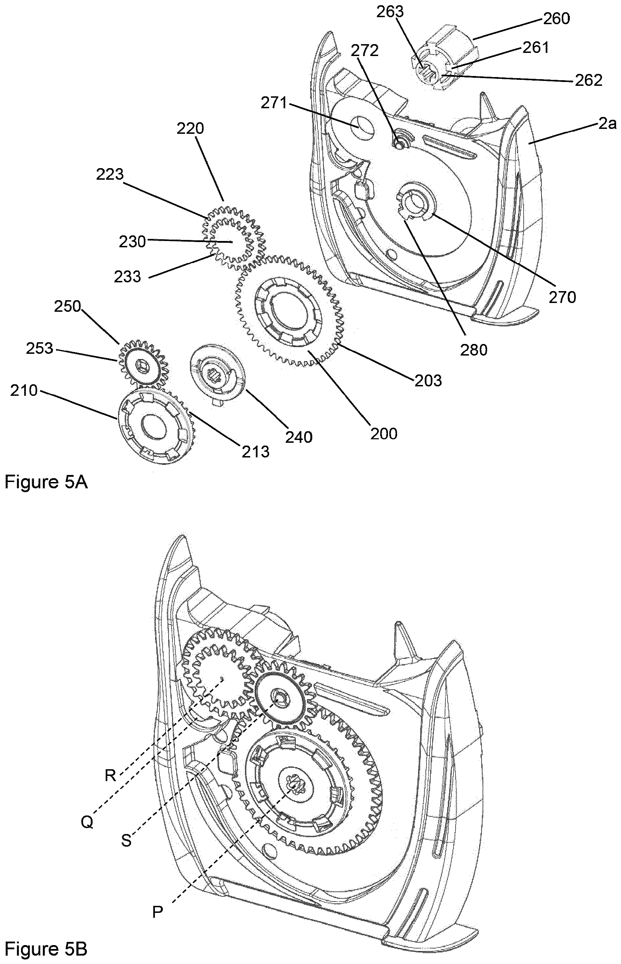

Figures 5 to 7 show the indexing mechanism of a second embodiment of an inhaler of the invention. In this embodiment, the opening and closing gears are situated on the same side of the housing and there is only one drive coupling, which couples to both the opening and closing drive gears (i.e. a dual drive coupling).

The dual drive coupling serves the same function as the separate drive couplings of in the first embodiment, i.e. to change the gearing between the outer cover and the indexing wheel from forward during the first part of the opening stroke, into neutral during the second part of opening and the first part of closing, and then into reverse during the second part of closing. However, the dual drive coupling performs this function in a different way, by selectively coupling the outer cover to the actuator gears instead of selectively coupling the drive gears to the indexing wheel. Having only one drive coupling has the advantage that there is no possibility of two drive couplings becoming out of synchronization, which could in principle occur in the first embodiment.

Figures 5A and 5B show expanded and assembled views of the mechanism, together with one shell portion of the housing **2a.** The mechanism has a first actuator gear **200,** a first drive gear **220,** a blister strip indexing wheel **260,** a second drive gear **230,** an idler gear wheel **250,** a second actuator gear **210** and a dual drive coupling **240** located between the first and second actuator gears. There are two important differences in the gears compared to the first embodiment.

Firstly, both drive gears are permanently coupled to the indexing wheel. Thus, as shown in Figure 5A, the first and second drive gears can be linked together or formed as a single component. Consequently, the gear train formed by the first actuator gear **200,** first drive gear **220,** indexing wheel **260,** second drive gear **230,** idler gear **250** and second actuator gear **210** is always connected. However, unlike the first embodiment, the actuator gears are not always coupled to the outer cover.

Secondly, each gear rotates in the same sense during both opening and closing, whereas in the first embodiment they rotate in one sense during opening and in the opposite sense during closing. Thus all of the gears must be full gear wheels, rather than just gear segments. In order that the angle through which the indexing wheel is the same on both opening and closing, the gear ratio between the actuator gear and drive gear must be the same for each pair. The gear ratio determines the angle through which the indexing wheel is rotated on each actuation.

As shown in Figure 5B, Axes **P, Q, R** and **S** are located in the same positions on the housing as in the first embodiment. An actuator gear axle **270** for mounting the first and second actuator gears and the drive coupling is located on axis **P.** A track formation **280** extends radially outwards from the axle **270.** A circular hole **271** in the housing provides a bearing surface for mounting the drive gears and indexing wheel on axis **R.** An idler gear wheel axle **272** is located on axis **S**.

The inner layer of the gear mechanism (adjacent to the housing) consists of the first actuator gear **200** and the first drive gear **220.** The first actuator gear **200** is mounted for rotation on the actuator gear axle **270.** The first drive gear **220** is mounted on the indexing wheel **260,** by means of a cruciform shaft **221** on the first drive gear wheel (not visible in this Figure) which extends through hole **271** and fits into a corresponding cruciform slot **263** inside the hub **262** of the indexing wheel **260.** The first drive gear **220** therefore rotates together with the indexing wheel **260** about axis **R.** The first actuator gear **200** engages with the first drive gear **220** via teeth **203, 223** on each wheel.

The outer layer consists of the second actuator gear **210,** the second drive gear **230** and the idler gear **250.** The second actuator gear **210** is co-axial with the first actuator gear and is also mounted for rotation about axis **P** on the actuator gear axle **270.** The second drive gear **230** is co-axial with the first drive gear **210** and is also mounted for rotation about axis **R.** The second drive gear may be formed (e.g. moulded) as a single component with the first drive gear, or may be a separate component which is fixedly mounted on the first drive gear. The second actuator gear **210** and second drive gear **230** have smaller radii than the first actuator gear **200** and drive gear **220** respectively. Consequently, their teeth **213, 233** do not engage with each other. However, the second actuator and drive gears both engage with the teeth **253** of the idler gear **250** which is mounted on the second hub **272** for rotation about axis **S.** Consequently, the second actuator and second drive gear rotate in the same sense as each other, and the opposite sense to the idler gear.

The drive coupling **240** is located between the first and second actuator gear wheels **200, 210.** The function of the drive coupling **240** is to selectively couple the outer cover to the first actuator gear **200** during the first part of opening, to uncouple the outer cover from both actuator gears during insertion and removal of the piercing elements and to couple the outer cover to the second actuator gear during the second part of closing. Since the first and second actuator gears rotate in opposite senses, rotating the outer cover in either direction (opening or closing) always causes the drive gears to rotate in the same direction, and hence the indexing wheel always moves the blister strip forwards.

Since the first and second drive gears are fixedly linked to each other (via the indexing wheel), the whole gear train (first actuator gear-first drive gear - indexing wheel - second drive gear - idler gear - second actuator gear) is always engaged (i.e. linked together). The first and second actuator gears rotate in opposite senses because of the idler gear.

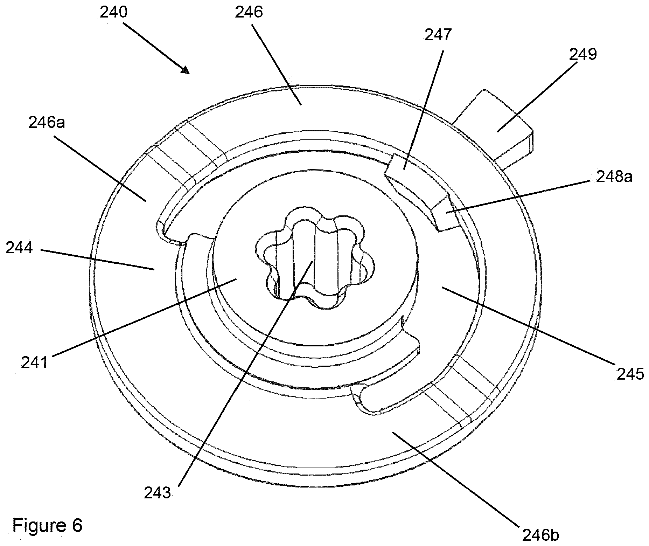

Figure 6A shows the drive coupling **240.** It has a hub **241** with a star-shaped central recess **243** and a circular flange **244** that extends radially from the hub **241.** A corresponding star-shaped peg on the inside of the outer cover fits into the central recess **243.** The outer cover is thereby fixedly linked to the drive coupling so that rotation of the outer cover causes the drive coupling to rotate.

The flange **244** has a cut-away arcuate opening **245** where it joins the hub **241** so that approximately half **246** of the flange **244** is not directly attached to the hub **241** but only to the other portion of the flange **244** at each of its ends **246a, 246b.** As a result, this portion **246** of the flange **244** is flexible and can be deflected out of the plane of the flange **244** towards the first or second actuator gear when force is applied to it. The flange **244** is made from a resilient material so that when the deflected flexible flange portion **246** is released, it returns to its neutral position, in which it is coplanar with the remaining fixed portion of the flange **244.**

The flexible flange portion **246** has an integrally formed flange deflecting dog **247** which projects radially inwards into the arcuate opening **245** mid-way along it. The flange deflecting dog **247** has first and second angled engaging faces **248a, 248b** on opposite ends, only one of which is visible in Figure 6. It functions in essentially the same way as the flange deflecting dog in the first embodiment. Opposite the flange deflecting dog, a drive dog **249** extends a short distance radially outwards from the outer edge of the flexible flange portion **246.** This drives both the first and second actuator gears, in a manner which will be described below.

The track formation **280** is rhomboid in cross-section, so that it has an angled engaging face **284a, 284b** at either end. These guide the flange deflecting dog **247** along one side of the track formation **280** as the outer cover is opened and back along the opposite side as it is closed, in the same manner as described above for the first embodiment. As the flange deflecting dog **247** engages with, and moves around, the track formation **280,** the flexible flange portion **246** is deflected so that the drive dog **249** engages and disengages with the spokes **204, 214** on the first and second actuator gears **200, 210** to drive them.

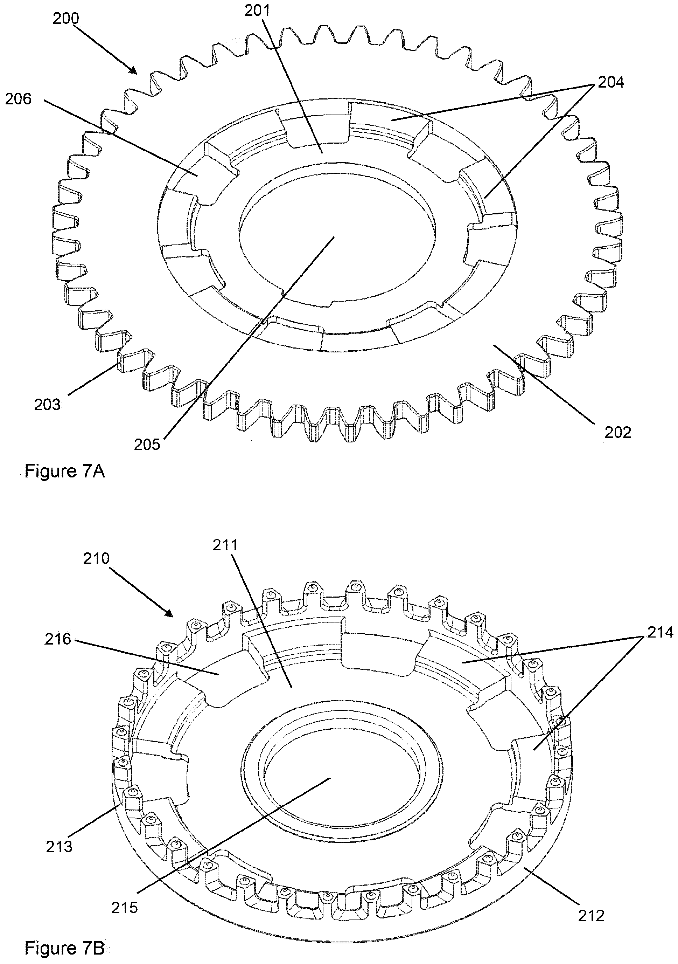

Figures 7A and 7B show the first and second actuator gears **200, 210** respectively, each viewed from the side adjacent to the drive coupling. The actuator gears **200, 210** are formed as wheels with a hub **201, 211,** and a rim **202, 212** from which the teeth **203, 213** protrude. The hub and rim are connected by spokes **204, 214.** Each hub **201, 211** has a central hole **205, 215** for mounting the actuator gear on the actuator gear axle **270.**

The drive dog **249** is displaced into a gap **206, 216** between two of the spokes **204, 214** when the flexible flange portion **246** has been deflected, so that the actuator gear **200, 210** is driven together with the drive coupling **240.**

When the outer cover is in the fully closed position, the flange deflecting dog **247** is adjacent to the first end of the track formation and lies in the same (neutral) plane. The drive dog **249** is also lies in this plane. As the outer cover is opened, the drive coupling is rotated so that the first angled engaging face **248a** of the flange deflecting dog **247** comes into contact with the first angled engaging face **284a** of the track formation **280.** Further rotation of the drive coupling **240** causes the flange deflecting dog **247** to ride along the angled engaging face **284a** to the inner side of the track formation **280** and along the first track. The drive dog **249** is also deflected into a gap **206** between two of the spokes **204** on the first actuator gear **200.**

Opening the outer cover further causes the drive coupling to rotate, so that the flange deflecting dog **247** rides along the inner side of the track formation **280.** The drive dog **249** comes into contact with the spoke **204** in front of it, so that the first actuator gear **200** is driven together with the drive coupling **240,** thereby causing the first drive gear and indexing wheel to rotate to move the blister strip as described above.

At the point when the flange deflecting dog **247** passes the end of the track formation **280,** it falls off. The resilience of the flexible flange portion **246** causes it to return to its neutral position, so that the drive dog **249** moves out of alignment with the spokes **204** of the first actuator gear **200.** The drive coupling thus returns to neutral. This is the end of the first part of the opening stroke of the outer cover. Further rotation of the outer cover through the second part of the opening stroke causes the flange deflecting dog **247** to rotate further, whilst remaining in the neutral plane. Since the drive coupling is disengaged, the blister strip is not indexed any further. The further rotation of the outer cover causes the piercing elements to pierce the aligned blisters, as described above.

After inhalation, the user closes the outer cover. Initially, the flange deflecting dog **247** travels back towards the track formation **280,** during which the piercing elements are removed from the pierced blisters while the drive coupling remains in neutral. The angled engaging faces **248b, 284b** deflect the flange deflecting dog **247** onto the other (i.e. outer) side of the track formation **280.** The drive dog **249** is deflected outwards into a gap **216** between two of the spokes **214** of the second actuator gear **210.**

As the outer cover continues to close, the drive coupling rotates, so that the flange deflecting dog **247** rides along the track formation **280.** The drive dog **249** comes into contact with the spoke **204** in front of it, thus driving the second actuator gear **210** and thereby indexing the blister strip forwards, as described above.

When the flange deflecting dog **247** passes the end of the track formation **280,** the resilience of the flexible flange portion **246** causes it to return to its neutral position, so that the drive dog **249** moves out of alignment with the spokes **214** of the second actuator gear **210.** The drive coupling thus disengages from the second actuator gear, so that it is in neutral when the outer cover is fully closed. Further actuations of the device repeat this cycle.

In the embodiment shown in Figures 5 to 7, each actuator gear **200, 210** has seven spokes **204, 214** and the indexing wheel **260** has four spokes **261.** The gear ratio between each actuator gear and its corresponding drive gear is chosen so that rotation of the actuator gear **200, 210** through 1/7^{th} of a circle (51.4°) causes the indexing wheel **260** to rotate through 90° in order to index the blister strip by one blister, i.e. a gear ratio of 7:4. The outer cover rotates through approximately 90° during opening. During the first few degrees, the drive coupling engages with the first actuator gear. Then, for the next 51.4° the drive coupling drives the first actuator gear and the blister strip is indexed. The final approximately 35° of rotation of the outer cover is used for piercing.

The gear ratio between the actuator gears and their corresponding drive gears can be chosen to index the blister strip by different amounts. For example, in order to move the strip by half a blister pitch or one and a half blister pitches, the gear ratios would be 7:8 and 21:8 respectively.

A different number of spokes could be chosen to adjust the amounts of the outer cover motion that drive indexing and piercing. The number of actuator gear spokes (N) determines the angle through which the actuator gear rotates (360°/N). For example, six spokes corresponds to 60° for indexing and 30° for piercing. There must be at least five spokes - if there were only four spokes, the whole 90° would be used for indexing, leaving nothing for piercing. Six or seven spokes has been found to be preferable. Higher numbers result in smaller gaps between the spokes, which means that there is less angular motion available for indexing, so the user must apply a greater torque.

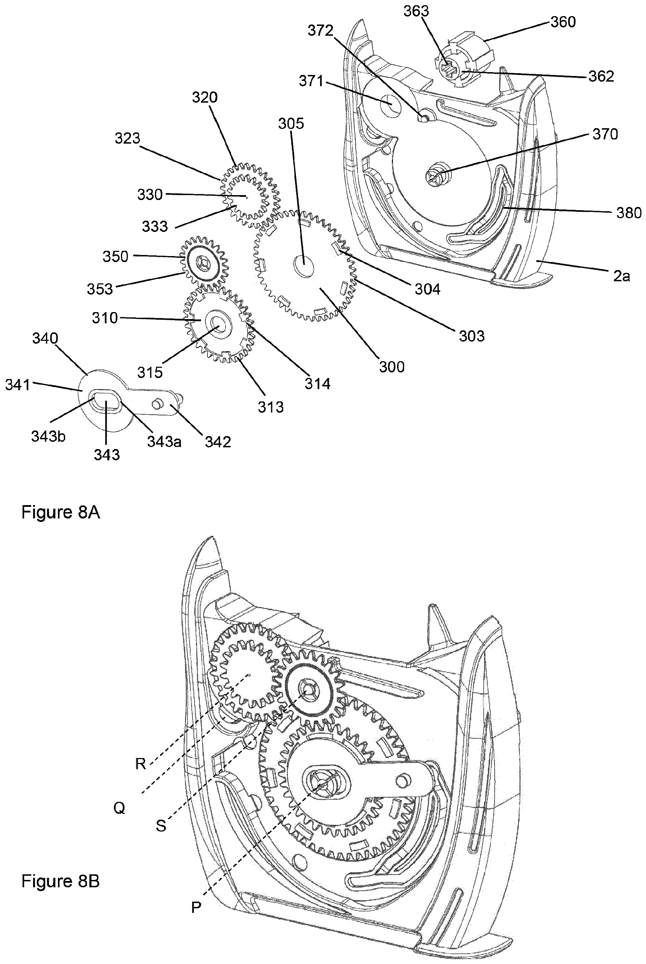

Figures 8 to 12 show the indexing mechanism of a third embodiment of an inhaler of the invention. The third embodiment is similar to the second embodiment, but the dual drive coupling takes a different form.

Figures 8A and 8B show expanded and assembled views of the actuating mechanism of the third embodiment, together with one shell portion of the housing **2a.** The mechanism has a first actuator gear **300,** a first drive gear **320,** a blister strip indexing wheel **360,** a second drive gear **330,** an idler gear wheel **350,** a second actuator gear **310** and a drive coupling **340** located on the outer side of gear mechanism.

As with the second embodiment, both drive gears are permanently coupled to the indexing wheel. Thus, as shown in Figure 8A, the first and second drive gears can be linked together or formed as a single component. The gear train formed by the first actuator gear **300,** first drive gear **320,** indexing wheel **360,** second drive gear **330,** idler gear **350** and second actuator gear **310**) is always connected. The drive coupling selectively couples the outer cover to the first or second actuator gear and each gear rotates in the same sense during opening and closing.

As shown in Figure 8B, axes **P, Q, R** and **S** are located in the same positions on the housing as in the previous embodiments. An actuator gear axle **370** for mounting the first and second actuator gears and the drive coupling is located on axis **P.** A circular hole **371** in the housing provides a bearing surface for mounting the drive gears and indexing wheel on axis **R.** An idler gear axle **372** is located on axis **S.** A track **380** is located on the housing to the side of the area occupied by the gear wheels. The track is described further below.

The inner layer of the gear mechanism (adjacent to the housing) consists of the first actuator gear **300** and the first drive gear **320.** The first actuator gear **300** is mounted for rotation on the actuator gear axle **370** by its central hole **305.** The first drive gear **320** is mounted on the indexing wheel **360,** by means of a cruciform shaft on the first drive gear wheel (not visible in this Figure) which extends through hole **371** and fits into a corresponding cruciform slot **363** inside the hub **362** of the indexing wheel **360** (also not visible). The first drive gear **320** therefore rotates together with the indexing wheel **360** about axis **R.** The first actuator gear **300** engages with the first drive gear **320** via teeth **303, 323** on each wheel. There are seven equi-angularly spaced actuator gear dogs **304** in the form of blocks which protrude from the outer surface of the first actuator gear **300** and which engage with the drive coupling in a manner that is described below.

The middle layer consists of the second actuator gear **310,** the second drive gear **330** and the idler gear **350.** The second actuator gear **310** is co-axial with the first actuator gear and is also mounted for rotation about axis **P** on the actuator gear axle **370** by its central hole **315.** The second drive gear **330** is co-axial with the first drive gear **320** and is also mounted for rotation about axis **R.** The second drive gear may be formed (e.g. moulded) as a single component with the first drive gear, or may be a separate component which is fixedly mounted on the first drive gear. The second actuator gear **310** and second drive gear **330** have smaller radii than the first actuator gear **300** and first drive gear **320** respectively. Consequently, their teeth **313, 333** do not engage with each other. However, the second actuator and drive gears both engage with the idler gear **350** which is mounted on the second hub **372** for rotation about axis **S.** Consequently, the second actuator and second drive gear rotate in the same sense as each other, and the opposite sense to the idler gear. There are seven equi-angularly spaced actuator gear dogs **314** in the form of blocks which protrude from the outer surface of the second actuator gear **310** which engage with the drive coupling as described below.

Since the first and second drive gears are fixedly linked to each other (via the indexing wheel), the whole gear train (first actuator gear - first drive gear - indexing wheel - second drive gear - idler gear - second actuator gear) is always engaged (linked together). The first and second actuator gears rotate in opposite senses because of the idler gear.

The drive coupling **340** is located on the outer side of the second actuator gear **310** (i.e. adjacent to the outer cover). The function of the drive coupling **340** is to selectively couple the outer cover to the first actuator gear during the first part of opening, to uncouple the outer cover from both actuator gears during insertion and removal of the piercing elements and to couple the outer cover to the second actuator gear during the second part of closing. Since the first and second actuator gears rotate in opposite senses, rotating the outer cover in either direction (opening or closing) always causes the drive gears to rotate in the same direction, and hence the indexing wheel always moves the blister strip forwards.

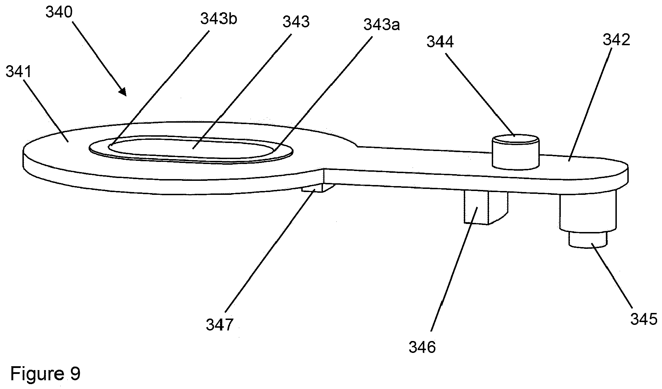

Figure 9 shows the drive coupling **340** which has a flange **341** and an arm **342.** The flange has a slot **343** with straight sides and rounded ends which mounts the drive coupling **340** on the actuator gear axle **370.** The slot **343** allows the drive coupling both to rotate about axis **P** and to translate radially between two drive positions. In the first drive position, the first rounded end **343a** of the slot **343** is in contact with the actuator gear axle **370,** so that the tip of the arm **342** is at its closest position to axis **P.** In the second drive position, the second rounded end **343b** of the slot is in contact with the axle **370,** so that the tip of the arm **342** is at its furthest position from axis **P.** In between these, when the axle **370** is intermediate between the two ends of the slot, the drive coupling **340** is disengaged, i.e. in neutral.

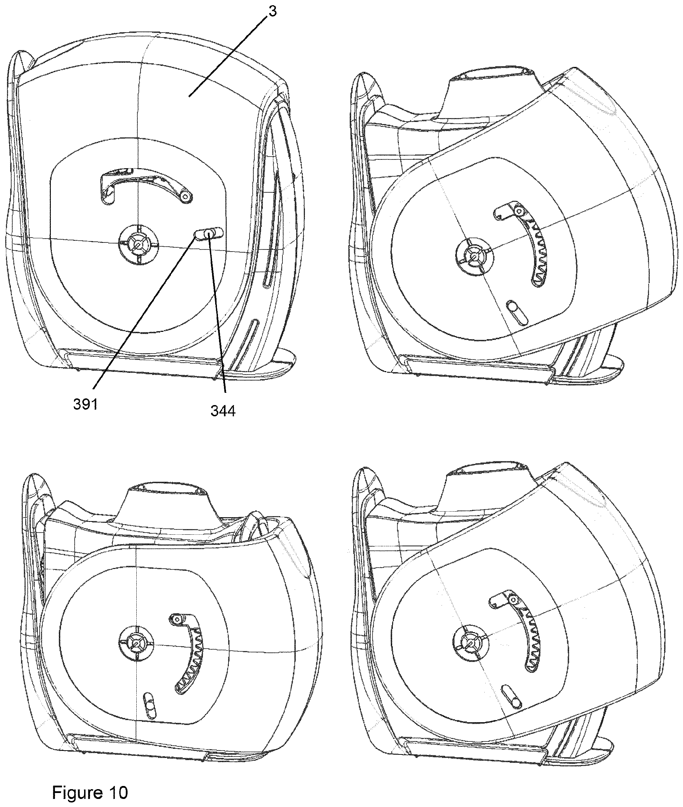

A drive pin **344** protrudes from the outer side of the arm. The drive pin **344** fits in a radial slot **391** in the outer cover **3,** as shown in Figure 10. The cover slot **391** is the same length as the slot **343** in the drive coupling **340.** Thus when the drive coupling **340** moves in the radial direction, the drive pin **344** also moves along the slot **391** in the outer cover **3.** Figure 10A shows the outer cover **3** in the closed position, so that the drive coupling **340** is neutral and the drive pin **344** is in the centre of the slot **391.** In Figure 10B, the outer cover is opening, so that the drive coupling **340** is in the first drive position and the drive pin **344** is at the inner end of the slot **391.** Figure 10C shows the outer cover **3** in the open position, so that the drive coupling **340** is back in neutral and the drive pin **344** is in the centre of the slot **391.** In Figure 10D, the outer cover **3** is closing, so that the drive coupling **340** is in the second drive position and the drive pin **344** is at the outer end of the slot **391.** Thus the cover slot **391** pushes against the drive pin **344,** and thereby causes the drive coupling **340** to rotate, whichever position the drive pin is in.

As also shown in Figure 9, the track follower **345** is located on the opposite (inner) side of the drive coupling **340,** near the tip of the arm **342.** The track follower **345** is a peg which moves around the track **380** on the housing as the outer cover is opened and closed, as will be described in detail below. First and second drive dogs **346, 347** are also located on the inner side of the drive coupling **340.** These engage with the actuator gear dogs **304, 314** on the first and second actuator gears when the arm **342** is in the first and second positions respectively, and thereby drive the actuator gears **300, 310,** as will also be described in detail below. The second drive dog **347** is shorter (i.e. protrudes a smaller distance from the drive coupling) than the first drive dog **346** because the second actuator gear **310** (with which the second drive dog **347** engages) is located closer to the drive coupling than the first actuator gear **300.**

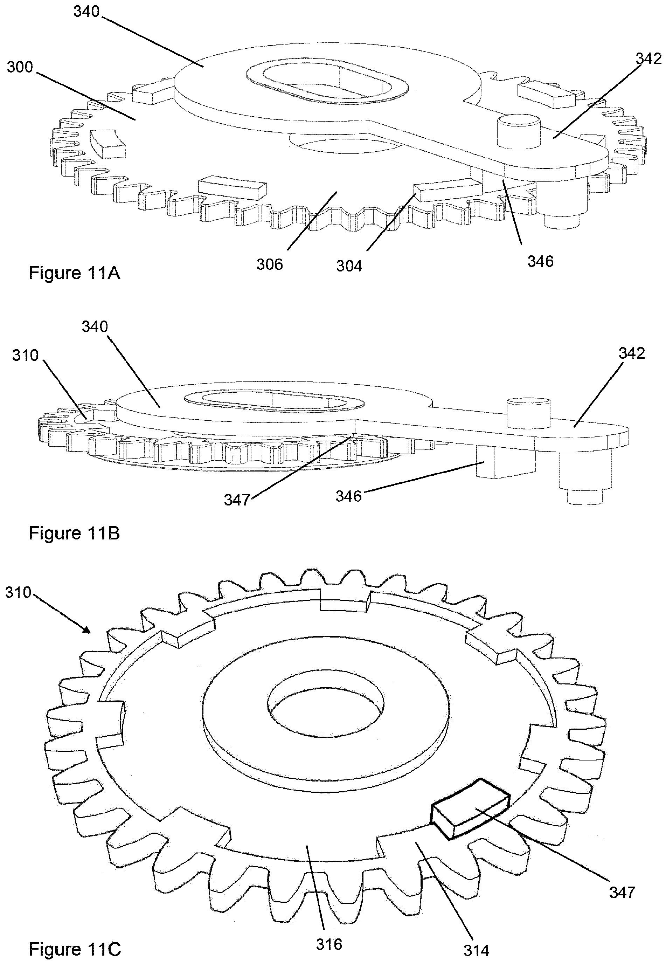

Figure 11A shows the drive coupling **340** in the first drive position and the first actuator gear (the axle and the second actuator gear, which lies between the drive coupling **340** and the first actuator gear **300,** are not shown in this Figure for clarity). In this position, the tip of the arm **342** is at its closest position to axis **P;** the first drive dog **346** is at the same radius as the first actuator gear dog **304,** and the second drive dog (not visible in Figure11A) is at a smaller radius than the second actuator gear dog **314.** Thus in the first drive position, rotation of the drive coupling **340** causes the first actuator drive dog **346** to push against the first actuator gear dog **304,** and hence to drive the first actuator gear **300.**

Figure 11B shows the drive coupling **340** and the second actuator gear **310** in the second drive position. In this position, the tip of the arm **342** is at its furthest position from axis **P** and the first drive dog **346** is at a larger radius than the first actuator gear dog **304.** The second drive dog **347** is mostly hidden Figure 11B, but is shown in Figure 11C in which the other parts of the drive coupling have been removed for illustration. The second drive dog **347** is at the same radius as the second actuator gear dog **314.** Consequently, in the second drive position, rotation of the drive coupling causes the second actuator drive dog **347** to push against the second actuator gear dog **314,** and hence to drive the second actuator gear **310.**

The actuator gear dogs therefore serve the same function as the spokes on the actuator gears in the previous embodiment.

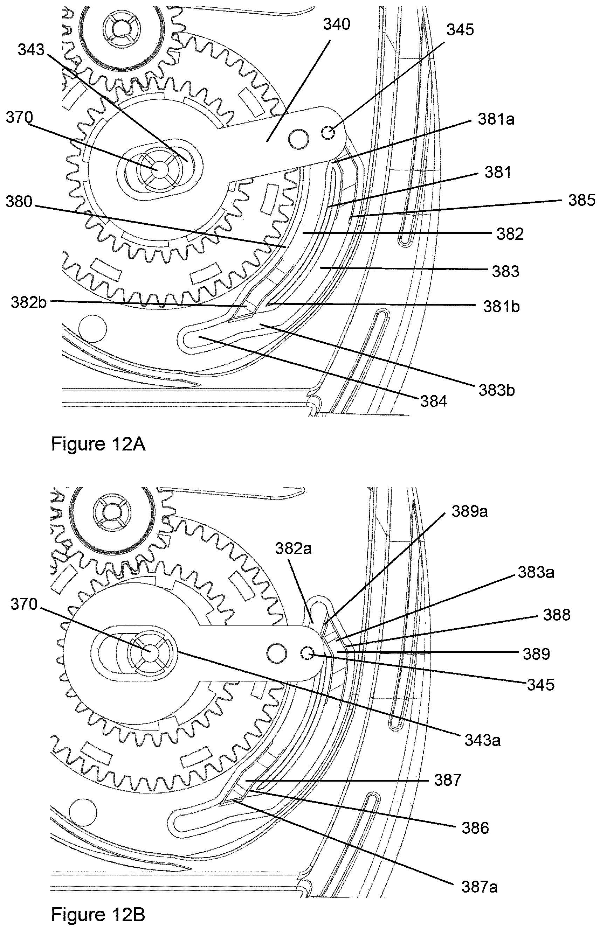

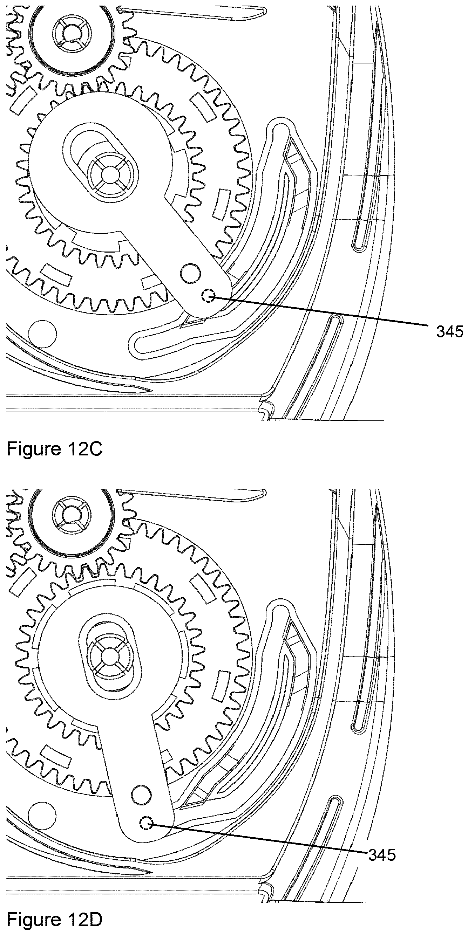

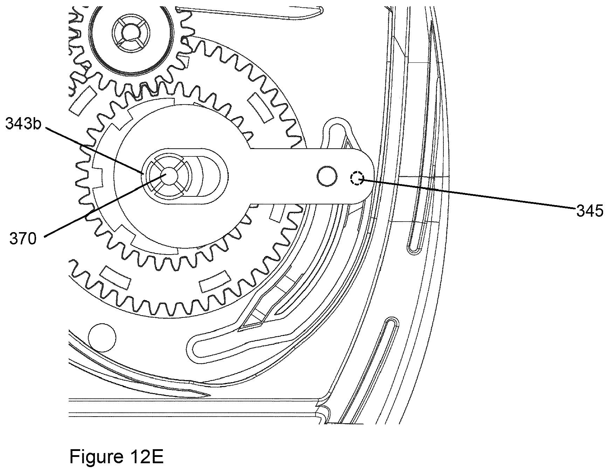

Figure 12 shows the drive coupling **340** in different positions around the track **380** during one full opening and closing cycle. The track has a central barrier **381** which lies between and separates a first (inner, opening) track portion **382** and a second (outer, closing) track portion **383.** Both track portions **382, 383** are arcs of circles centered on axis **P.** The radius of the outer track portion **383** is equal to the radius of the inner track portion **382** plus the length of the drive coupling slot **343.** Both the inner and outer tracks have short straight sections at each end **382a, 382b, 383a, 383b.** These meet at a radius halfway between the radii of the inner and outer track portions. At one end, a neutral (piercing) track portion **384** extends beyond the ends of the inner and outer tracks **382b, 383b** from the point at which the straight sections meet. The central barrier **381** tapers to a point at each end **381a, 381b.** A wall **385** surrounds the track portions **382, 383, 384.**

The inner track **382** has a cut-away opening **386** which extends from approximately the mid-point to its second end **382b** along both sides (i.e. adjacent to the central barrier **381** on one side and adjacent to the wall **385** on the other), and across the second end **382b** of the inner track, in line with the tapered outer side of the central barrier **381.** The cut-away opening defines a portion **387** of the inner track which is not directly attached to the housing on three sides and which can be deflected when force is applied to it. The track **382** is made from a resilient material so that when the deflected flexible portion **387** is released, it returns to its original position. The flexible portion **387** therefore acts as a leaf spring. Moreover, the flexible portion is shaped so that it slopes outwards (i.e. out of the plane of the paper in Figure 12) from the point where is joined to the inner track to its free end. The end of the flexible portion **387** consequently forms a protruding lip **387a.** The outer track **383** similarly has a cut-away opening **388** from approximately its mid-point to its first end **383a** which forms a flexible portion **389** with a lip **389a.**

Figure 12A shows the configuration when the outer cover is fully closed. The track follower **345** is hidden beneath the arm of the drive coupling in this view; however, its position is indicated in Figure 12 by a dashed circle. The track follower **345** is at the point where the straight sections of the inner and outer tracks meet at their first ends **382a, 383a,** i.e. at the intermediate radius, so the drive coupling is in the position where the actuator gear axle **370** is at the mid-point of the slot **343.** In this position, the drive dogs **346, 347** are not at the same radius as their corresponding actuator gear dogs **304, 314** so the drive coupling **340** is in neutral.

As the outer cover is opened, the track follower **345** initially moves along the inner short straight section at the first end of the inner track **382a.** The lip **389a** guides the track follower **345** onto the inner track **382** and ensures that it does not go onto the outer track **383.** The track follower **345** then contacts the tapered portion **381a** of the central barrier **381,** which pushes the track follower **345,** and hence the drive coupling **340** radially inwards until the first end **343a** of the slot **343** abuts the actuator gear axle **370.** The first drive dog **346** passes into one of the gaps **306** between the actuator gear dogs **304** on the first actuator gear **300.** The second drive dog **347** is located at a smaller radius than the second actuator gear dogs **314,** so cannot come into contact with them. This configuration is shown in Figure 12B.

Opening the outer cover further causes the drive pin **344** and drive coupling **343** to rotate. The track follower **345** is confined by the central barrier **381** and surrounding wall **385** to move along the inner track **382.** The first drive dog **346** is at the same radius as the first actuator gear dogs **304** and comes into contact with the adjacent one. This drives the first actuator gear **300,** thereby indexing the blister strip, as described above.

Figure 12C shows the configuration at the point at which the track follower **345** approaches the end of the inner track **382a.** This corresponds to the end of the first part of the opening stroke of the outer cover. The track follower **345** pushes the flexible portion **387** inwards (i.e. into the plane of the paper in Figure 12) so that the flexible portion **387** is level with the rest of the track. The track follower **345** thereby passes over the flexible portion **387.** At the same time, the surrounding wall **385** of the short straight section guides the track follower **345** and hence the drive coupling **340** radially outwards to a neutral position at a radius intermediate between the inner and outer arcuate track portions. Once the track follower **345** has passed over the flexible portion **387** it moves onto the piercing track **384** and the flexible portion **387** springs back to its original position.

At the end of the inner track portion **382b,** the actuator gear axle **370** is halfway along the slot **343** in the drive coupling **340** and the first drive dog **346** has been moved out of radial alignment with the first actuator gear dogs **304.** The drive coupling **340** is thus in neutral. Further rotation of the outer cover (though the second part of the opening stroke) causes the track follower **345** to move along the piercing track **384.** Since the drive coupling **340** is in neutral, the outer cover is not connected to the gears so the blister strip is not indexed any further. However, the rotation of the outer cover through the second part of the opening stroke motion causes the piercer to pierce the aligned blisters, as described above. Figure 12D shows the configuration at the point at which the outer cover is fully open and the piercing elements have entered the aligned blisters.

After inhalation, the user closes the outer cover. Initially, the track follower **345** travels back along the piercing track **384,** during which the piercer is removed from the pierced blisters while the drive coupling remains in neutral. Instead of returning along the inner track **381,** the raised lip **387a** of the flexible portion **387** of the inner track **382** guides the track follower **345** to the outer side of the central barrier **381** and onto the outer track **384.** In an analogous manner to that described above with reference to Figure 12B and 12C, the drive coupling **340** is pushed radially outwards until the second end of the slot **343b** abuts the actuator gear axle **370.** The second drive dog **347** moves radially outwards into a gap **316** between two of the second actuator gear dogs **314,** so that it is at the same radius. The first drive dog **346** is now located at a larger radius than the first actuator gear dogs **304,** so cannot come into contact with them.

Further closing the outer cover causes the track follower **345** to move along the outer track **383** and the second drive dog **347** to contact the adjacent second actuator gear dog **314,** thereby causing the second actuator gear **310** to rotate. This in turn indexes the blister strip forwards. Figure 12E shows the configuration when the track follower **345** approaches the end of the outer track **383.**

In the final part of the closing of the outer cover, the track follower **345** pushes inwards on the flexible portion **389** so that it is level with the rest of the track. After the track follower **345** has passed over the lip **389a** of the flexible portion **389,** the flexible portion **389** springs back to its original position. At the same time, the track follower **345** moves along the short straight section at the end **383a** of the outer track **383** which causes the second drive dog **347** to disengage from the second actuator gear dogs **314** so that the drive coupling **340** has returned to neutral. With the outer cover in the fully closed position, the drive coupling has returned to the initial, neutral position (Figure 12A). Further operations of the device repeat this cycle.

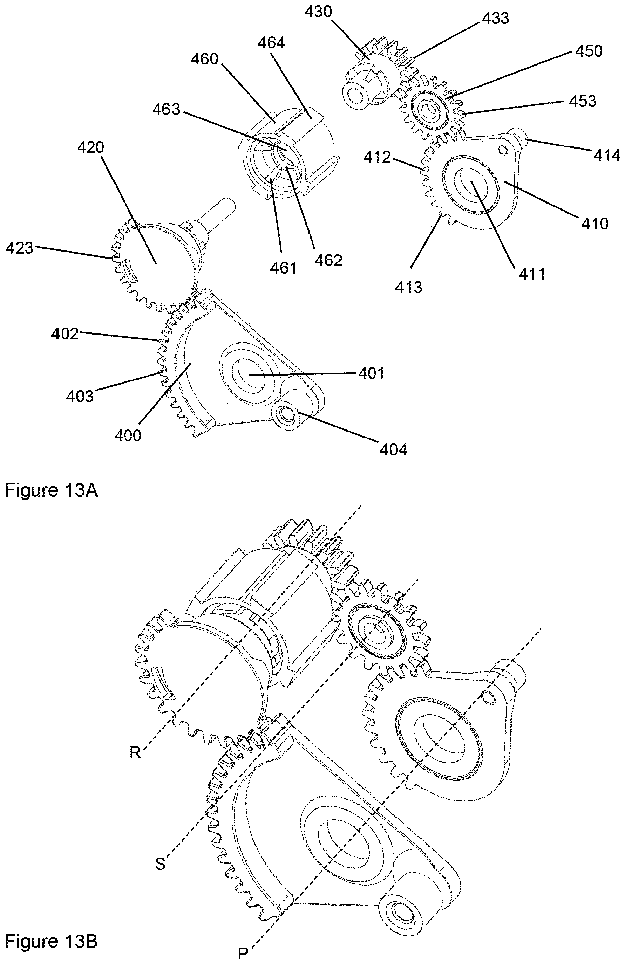

Figures 13 to 16 show a fourth embodiment, in which (like the first embodiment), the opening and closing gears are situated on opposite sides of the housing. Similarly, the actuator gears are always coupled to the outer cover and the drive gears are selectively coupled to the indexing wheel. However, in this embodiment there are no drive couplings between the drive gears and the indexing wheel. Instead, the drive gears themselves engage and disengage with the indexing wheel in a manner which is described below.

Figures 13A and 13B show expanded and assembled views of the actuating mechanism of the fourth embodiment. The mechanism is similar to that of the first embodiment, comprising a first (opening) actuator gear **400,** a first drive gear **420,** a blister strip indexing wheel **460,** a second drive gear **430,** an idler gear wheel **450** and a second (closing) actuator gear **410.** However, unlike the first embodiment, there are no drive couplings.

The first and second actuator gears **400, 410** are formed as plate-like portions, each having a central pivot hole **401, 411** which fit onto the axles **7** (see Figure 1) on either side of the housing, so that the actuator gears are mounted for rotation on the housing about the same axis **P** as the outer cover. The actuator gears **400, 410** each have gear elements **402, 412** which consist of teeth **403, 413** extending around part of the periphery of each gear.

The first and second actuator gears **400, 410** may be keyed or otherwise attached to the outer cover when the inhaler is assembled so that the first and second actuator gears and the outer cover rotate together. For example, round posts **404, 414** protrude from the actuator gears **400, 410** and are received in corresponding holes **15** in each side of the outer cover (see Figure 1). Opening or closing the outer cover thereby causes the actuator gears **400, 410** to rotate about axis **P.** Alternatively, the first and second actuator gears **400, 410** and outer cover may be formed as a single component, for example they may be moulded together as a unitary piece.

The first and second drive gears **420, 430** and the indexing wheel **460** are mounted for rotation about axis **R.** The indexing wheel **460** comprises a number of spokes **461** (typically four) extending from a hub **462** which surrounds a central circular recess **463.** The spokes are arranged so that a blister locates between the protruding ends **464** of successive spokes as the blister strip passes around the indexing wheel.

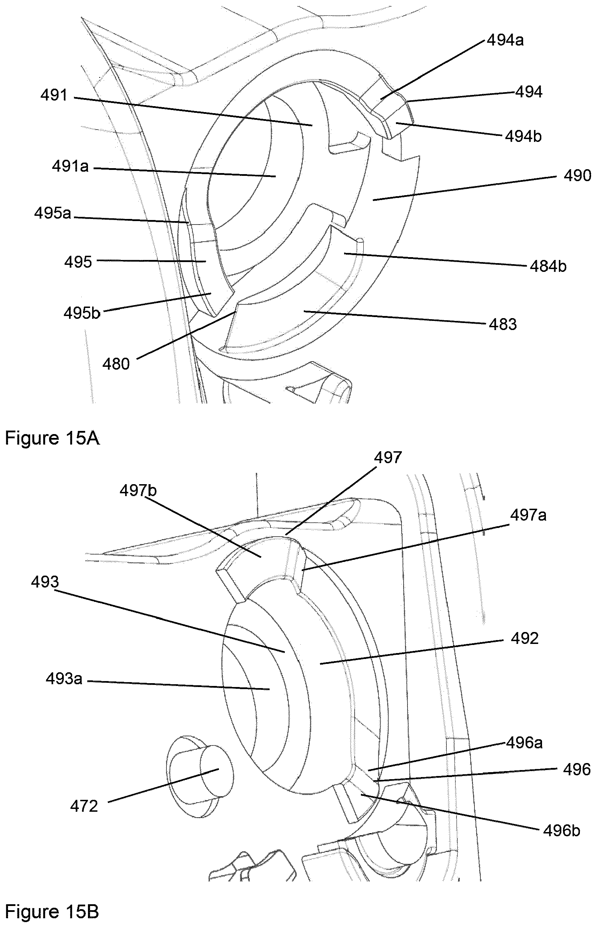

The first actuator gear element **402** transmits drive from the outer cover as it is rotated to the first drive gear **420** by means of the gear teeth **403** which mesh with corresponding gear teeth **423** on the first drive gear **420.** The second actuator gear element **412** transmits drive from the outer cover as it is rotated via the idler gear wheel **450** to the second drive gear **430.** The idler gear wheel is mounted for rotation on an idler gear axle **472 (shown in** **Figure 15B****)** about axis, **S.** The gear teeth **413** of the actuator gear element mesh with corresponding gear teeth **453** on the idler gear wheel which in turn mesh with gear teeth **433** on the second drive gear **430.**

Thus, as with the first embodiment, the first drive gear **420** rotates in response to rotation of the first actuator gear **400,** and the second drive gear **430** rotates in response to rotation of the second actuator gear **410**, but in the opposite sense, due to the presence of the idler gear wheel **450.** The drive gears **420, 430** and rotate together with their respective actuator gear **400, 410** at all times. The difference over the first embodiment is the manner in which the drive gears **420, 430** selectively connect to and disconnect from the indexing wheel **460,** in order to cause the indexing wheel **460** to rotate to index the blister strip.

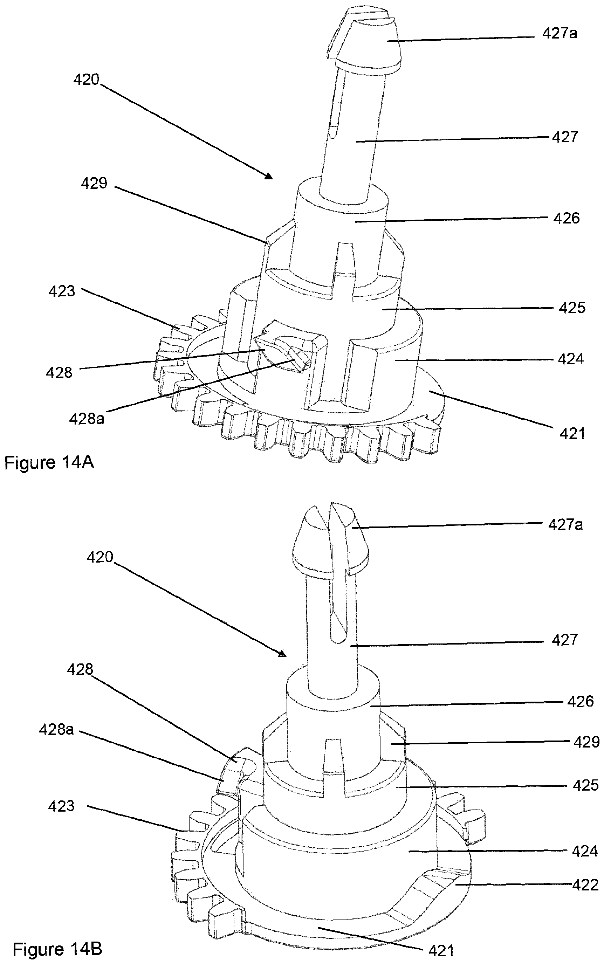

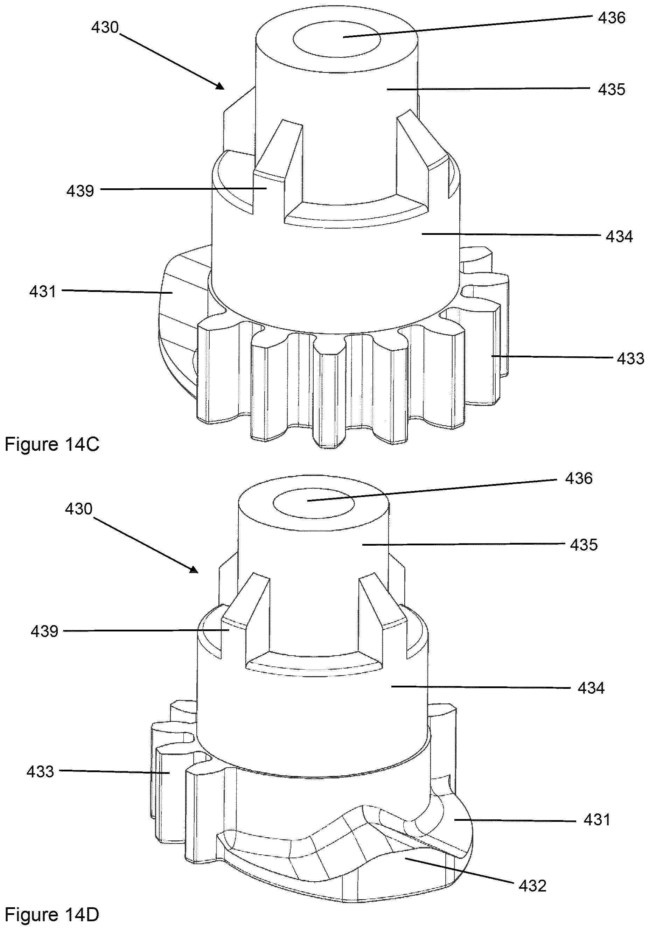

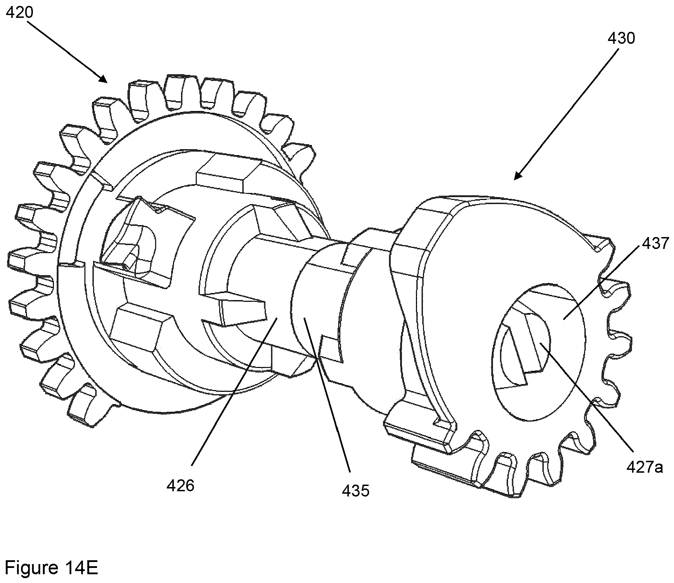

Figures 14A and 14B show perspective views from opposite sides of the first drive gear **420.** Figures 14C and 14D show similar views of the second drive gear **430.** Figure 14E shows the first and second drive gears connected together, without the indexing wheel in order to show the features that would otherwise not be visible.

The first drive gear **420** comprises a flange **421** having a small hump on one side which functions as a first ramp follower **422,** and a shaft which extends axially from the centre of the flange **421.** Gear teeth **423** protrude radially from the flange around about half of its circumference on the side opposite the hump. The shaft has first, second, third and fourth cylindrical sections **424, 425, 426, 427** having different radii. The first shaft section **424,** which has the largest radius, is adjacent to the flange. The fourth shaft section **427,** which is furthest from the flange, has the smallest radius. The second **425** and third **426** shaft sections lie between the first **424** and fourth **427** shaft sections, and have intermediate radii. A track follower **428** protrudes radially from the first shaft section **424** at approximately half way (axially) along it, diametrically opposite to the hump **422.** The track follower **428** has first and second angled engaging faces **428a, 428b** on its ends, so that it is rhomboid in shape (when viewed in the radial direction).

The second drive gear **430** comprises a flange **431** also having a hump which functions as a second ramp follower **432,** and an axial shaft. Gear teeth **433** protrude radially from the shaft around about half of its circumference on the side opposite the flange **431** and hump **432.** The shaft has first and second cylindrical sections **434, 435** with the same radii as the second **424** and third **425** shaft sections respectively of the first drive gear **420.** A central circular hole **436** extends through the drive gear **430.**

The fourth shaft section **427** of the first drive gear has a radius which corresponds to that of the central circular hole **436** of the second drive gear. A clip connection **427a** is situated at the distal end of the fourth shaft section **427** and fits into a corresponding circular recess **437** on the outer side of the second drive gear **430,** which is visible in Figure 14E.

Figure 14E shows the second drive gear **430** connected to and mounted for rotation on the fourth shaft section **427** of the first drive gear **420.** The clip connection **427a** and corresponding recess **437** hold the first **420** and second **430** drive gears together in the axial direction, whilst they are free to rotate independently of each other. The length of the fourth shaft section **427** (including the clip connection **427a**) corresponds to the total thickness of the second drive gear **430.** Thus, when the fourth shaft section **427** is inserted into the central circular hole **436,** the third shaft section **426** of the first drive gear **420** abuts the second shaft section **435** of the second drive gear **430.**

The radius of the third shaft section **426** of the first drive gear and of the second shaft section **435** of the second drive gear correspond to that of the central circular recess **463** of the indexing wheel **460.** These shaft sections **426, 435** abut each other and together form a further bearing surface (visible in Figure 14E) on which the indexing wheel **460** rotates, also on axis **R.**

Four equi-angularly spaced drive dogs **429, 439** protrude radially outwards from the third shaft section **426** of the first drive gear and from the second shaft section **435** of the second drive gear. The drive dogs **429** on the first drive gear are formed as walls which extend axially from the second shaft section **425** along part of the third shaft section **426.** Similarly, the drive dogs **439** on the second drive gear are formed as walls which extend axially from the first shaft section **434** along part of the second shaft section **435.**

The drive dogs engage and disengage with the spokes of the indexing wheel in a manner which is described below. The axial faces of both the drive dogs and the spokes are sloped at an angle of 45°. This maximizes the contact area between the radial faces of the drive dogs and the spokes when they are engaged for the fixed axial engagement distance, which is limited by the distance that the drive gears translate as will be described below.

Figures 15A and 15B show close up views of the regions of the first (front) and second (rear) sides of the housing where the drive gears are located. The first side of the housing has a first circular recess **490** within which a first ring **491** is formed. Correspondingly, the second side of the housing has a second circular recess **492** a second ring **493.** The radius of each ring **491, 493** corresponds to that of the second shaft section **425** of the first drive gear and the first shaft section **434** of the second drive gear.

The radially inward surface **491a** of the first ring **491** thereby forms a bearing surface for the second shaft section **425** of the first drive gear which mounts the first drive gear for rotation about axis R. Similarly, the corresponding radially inward surface **493a** of the second ring **493** forms a bearing surface for the first shaft section **434** of the second drive gear. The indexing wheel **460** is mounted for rotation about axis R on the bearing surface formed by the third shaft section **426** of the first drive gear **420** and the second shaft section **435** of the second drive gear **430.** At the same time, it is held in place axially in the housing between the inner axial faces of the rings **491, 493** (these faces are not visible in Figures 15A and 15B).

As shown in Figure 15A, a track formation **480** protrudes from the first circular recess **490,** in the form of a barrier which separates and defines first and second tracks **482, 483** on either side, in the same way as the first embodiment. The ends of the barrier have angled faces **484a, 484b.** Two ramps, a short close ramp **494** and a long pierce ramp **495,** protrude from the outer side of the housing adjacent to and on opposite sides of the circular recess **490.**

The second side of the housing, shown in Figure 15B, has similar close and pierce ramps **496, 497** but does not have a track formation. The ramps correspond in size and shape to the ramps **494, 495** on the front side of the housing, but are located at different angular positions around the circular recess **492.**

The track formation **480** on the first circular recess **490** and the track follower **428** on the first drive gear **420** operate in a similar manner to the first embodiment, but with some differences.

Firstly, unlike the first and second embodiments, there are no flexible portions that are deflected to cause engagement with the indexing wheel **460.** Instead, the first and second drive gears themselves translate axially with respect to the indexing wheel **460** along axis R so that the drive dogs **429, 439** engage with the spokes **461** of the indexing wheel. The drive gears are able to translate axially because the second shaft section **425** of the first drive gear and the first shaft section **434** of the second drive gear can slide axially on the bearing surfaces **491a 493a.** The distance that the drive gears translate is equal to the width of the track formation **480** plus the width of the track follower **428.** The absence of flexible parts has the advantage that the mechanism is robust and simple to manufacture.

Secondly, in order for the first and second drive gears to remain engaged with the first actuator gear and the idler gear respectively when they are translated, one gear of each pair is thicker, by an amount corresponding to the axial distance that the drive gears translate. As shown in Figures 13 and 14, the gear teeth **403, 433** of the first actuator gear **400** and the second drive gear **430** are thicker (axially) than the gear teeth **453, 423** of the idler gear **450** and first drive gear **420** (however, the thicker and thinner teeth could equally be the other way around). In order to ensure that the gears are fully engaged in both drive positions, the thickness of the thicker gears is thickness of the thin gear plus the distance that the drive gears translate.

The third difference is the mechanism by which return to the neutral position is achieved. In contrast to the first embodiment, there is no flexible portion whose resilience causes it to spring back to its neutral (undeflected) position once the track follower has passed the central barrier. Instead, return to the neutral position is achieved by the ramps and ramp followers in the manner described below.

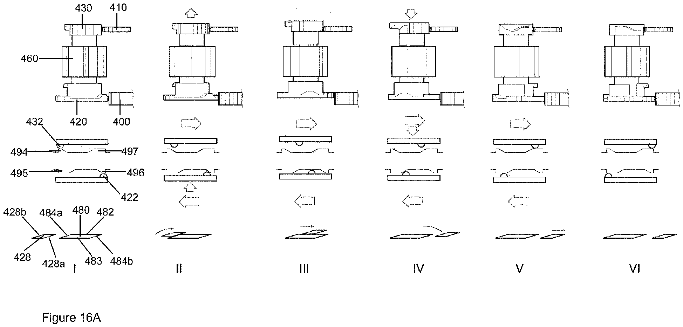

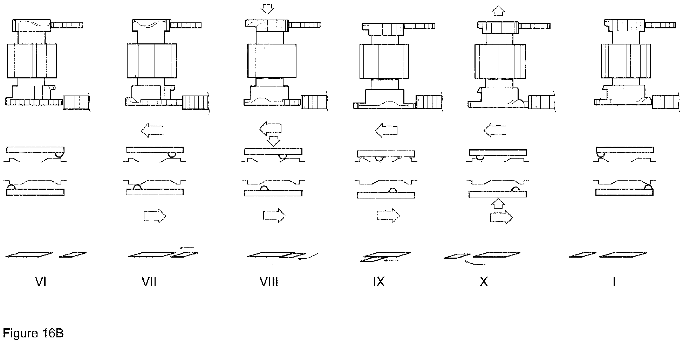

Figure 16 shows the positions of the actuator gears **400, 410** drive gears **420, 430** and the indexing wheel **460** (top row), the ramps **494, 495, 496, 497** and ramp followers **422, 432** and (middle row) and the track follower **428** and the track formation **480** (bottom row) at several stages during opening (Figure 16A) and closing (Figure 16B) of the outer cover. The track follower and ramp followers each move in a circular arc as the gears are rotated, but in Figure 16, the motion is schematically shown as being linear for simplicity.

In stage I, the outer cover is in the closed position. The gear mechanism is in neutral, and the drive gears are located centrally with respect to the indexing wheel. Stages II, III and IV correspond to the first part of the opening stroke. The drive gears move axially relative to the indexing wheel in a manner that is described in detail below, so that the first drive gear engages with the spokes of the indexing wheel **460.** Stage V is the second part of the opening stroke in which the first drive gear disengages from the indexing wheel and the gear mechanism is in neutral whilst the piercing elements are inserted. In stage VI, the outer cover is in the fully open position. Stage VII is the first part of the closing stroke in which the gear mechanism is in neutral whilst the piercing elements are removed. Stages VIII, IX and X correspond to the second part of the closing stroke. The drive gears move in the axially opposite direction, so that the second drive gear engages with the indexing wheel. Finally, at the end of the closing stroke, the drive gears return to the neutral central position and the cover returns to the fully closed position of stage I.

The mechanism operates as follows. When the outer case is in the closed position (stage I), the drive dogs **429, 439** on both the first and second **420, 430** drive gears are spaced apart from the spokes **461** of the indexing wheel **460.** The track follower **428** on the first drive gear **420** is spaced apart from the track formation **480.** The ramp followers **422, 432** sit on the tops of their respective close (short) ramps **494, 496.**

When the user opens the outer cover is opened, the gears rotate. As indicated by the arrows in Figure 16, the track follower **428** moves towards the track formation **480,** so that the first angled engaging face **428a** on the track follower comes into contact with the first angled engaging face **484a** of the track formation **480.** The track follower **428** rides up the angled engaging face **484a** (stage II) to one side **482** of the track formation. This pulls the first drive gear **420** inwards towards the indexing wheel **460** as the ramp follower **422** on the first drive gear moves down the sloping section **494a** of the first close ramp **494.** The second drive gear (which is connected to the first drive gear by the clip connection **427a**) correspondingly moves outwards and its ramp follower **432** moves clear of its close ramp **496.** As the first drive gear moves inwards, the drive dogs **429** engage the spokes **461** of the indexing wheel **460.** Consequently, as the track follower **428** moves along the first track **482** (stage III), the first drive gear **420** drives the indexing wheel **460** to rotate.

At the point when the track follower **428** reaches the end of the track formation **480** (stage IV), the ramp follower **422** on the first drive gear comes into contact with its long ramp **495.** Further rotation of the outer cover, and hence the drive gears, causes the ramp follower **422** to ride up the angled section **495a** of the pierce ramp **495.** This pushes the first drive gear outwards so that the drive dogs **429** disengage from the spokes **461** of the indexing wheel, and pulls the second drive gear **430** inwards. The first ramp follower **422** reaches the flat section of the pierce ramp **495b,** at which point the drive gears have returned to the central, neutral position. The ramp follower **432** on the second drive gear also comes into contact with the flat section **497b** of the second pierce ramp **497** on the second (rear) side of the housing. This contact prevents the drive gears from overshooting the neutral position. This is the end of the first part of the opening stroke.

As with the first embodiment, the length of the track formation **480** controls the extent of rotation of the indexing wheel **460** relative to the extent of rotation of the drive gears **420, 430.** Thus where two blisters are indexed and pierced on each actuation, the track length is chosen so that the indexing wheel is rotated through the correct angle to move the next, unused blister, into alignment with the blister piercing element during the opening stroke.

When the outer cover is rotated further during the second part of the opening stroke, drive to the indexing wheel is disengaged while the ramp followers **422, 432** move along the flat second sections **495b, 497b** of their respective pierce ramps until the outer cover reaches the fully open position. This second part of the stroke allows the blisters to be pierced while the indexing wheel is not being driven and the blister strip is stationary, in the same manner as before In the fully open position (stage VI), the piercing elements have been inserted and the ramp followers **422, 432** are at the far ends of the flat sections of the pierce ramps **495b, 497b.**

When the user closes the outer cover from the fully open position, the drive gears rotate back in the opposite directions. In the first part of the closing movement, the piercing elements are removed from the blisters and the ramp followers **422, 432** move back along the flat sections of the pierce ramps **495b, 497b** while the drive gears remain in the central, neutral position (stage VII).

At the start of the second part of the closing movement, the second angled engaging face **428b** of the track follower **428** contacts the second angled engaging face **484b** of the track formation **480** (stage VIII) and the ramp followers **422, 432** reach the start of the sloping sections **495a, 497a** of the pierce ramps.

Further rotation of the outer cover, and hence the first drive gear causes the track follower **428** to ride along the angled engaging face **484b** and move onto the second track **483** (Stage IX). The first drive gear moves outwards and the second drive gear moves inwards towards the indexing wheel. The ramp follower **432** on the second drive gear moves down the sloping section **497a** of the close ramp **497.** The other ramp follower **422** moves clear of its pierce ramp **495.** As the second drive gear moves inwards, its drive dogs **439** engage the spokes **461** of the indexing wheel **460.**

The indexing wheel **460** is now driven by the second drive gear **430** while the track follower moves along the second track **483** (stage IX). Since the second drive gear rotates in the opposite sense to the first drive gear due to the idler gear, the blister strip is indexed forwards (not backwards) by the closing of the outer cover, as in the first embodiment.

At the point when the track follower reaches the end of the track formation **480** (stage X), the ramp follower **432** on the second drive gear comes into contact with the close ramp **496** on the second (rear) side of the housing. Further rotation of the outer cover, and hence the drive gears causes the ramp follower **432** to ride up the angled section **496a** of the ramp **496.** This pushes the second drive gear outwards so that the drive dogs **439** disengage from the spokes **461** of the indexing wheel, and pulls the first drive gear **430** inwards.

The second ramp follower **432** reaches the top of the close ramp **496b,** at which point the drive gears have returned to the central, neutral position. The ramp follower **422** on the first drive gear also comes into contact with the top **494b** of the first close ramp **494.** This contact prevents the drive gears from overshooting the neutral position. This is the end of the closing stroke, and the mechanism has returned to its initial, fully closed position (stage I).

Thus the linked drive gears act as a shuttle, which is driven axially by the track formation, the track follower, the ramps and the ramp followers. The motion of the shuttle changes the gearing between the outer cover and the indexing wheel from forward during the first part of the opening stroke, into neutral during the second part of the opening stroke and the first part of the closing stroke, and then into reverse during the second part of closing. Consequently the blister strip is indexed forwards during both opening and closing.

Although the gear mechanisms are different, the four embodiments described above have a number of common features and advantages.

Firstly, all of the mechanisms are in a stable, neutral configuration when the outer cover is in the closed position. None of the components is under stress in this position; furthermore, if an external force is applied to the device, for example by dropping it, the gear mechanism remains in neutral and cannot be accidentally forced into a drive position.

Secondly, actuation of the inhaler is reversible during the first part of the opening stroke, up to the point at which the flange deflecting dog / track follower passes the end of the track formation / central barrier. The user can abort actuation simply by closing the outer cover, which moves the blister strip back to its previous position. Moreover, the closing stroke is reversible through both the first part when the piercing elements are removed and through most of the second part during which the blister strip is indexed, i.e. up to the point at which the flange deflecting dog / track follower passes the other end of the track formation / central barrier. Thus, for example, if the user accidentally begins to close the outer case without having inhaled, they can simply open it again, inhale and then close as normal.

Thirdly, each of the embodiments can straightforwardly be re-configured into a conventional inhaler that delivers the contents of one blister on each actuation, either by modifying the blister strip or by omitting some components of the device. Thus, by using a strip in which each blister is each half-filled, or with alternating full and empty blisters, a single dose is dispensed when two blisters are indexed and pierced on each actuation. Alternatively, omitting the second piercing element, second actuator gear, second drive gear and idler wheel provides an inhaler which indexes on opening only and pierces one blister on each actuation. Thus, a single overall design with one set of components can provide both conventional, and double indexing inhalers

Instead of causing indexing and piercing, the outer cover could alternatively be passive, so that opening or closing it exposes or covers the mouthpiece, but does not actuate the device. In this case, the inhaler includes a separate actuating lever which is revealed when the outer cover is rotated out of its closed position. The lever may have a protruding button to facilitate actuation by the user, as described in, for example, WO 13/175176. The actuating lever is connected to the actuator gears in an analogous manner to the outer cover in the embodiments described above. Thus in this variant of the first and fourth embodiments, in which the drive gears selectively engage and disengage with the indexing wheel, the actuating lever (instead of the outer cover) is keyed or otherwise attached to the outer cover, and indeed may be formed (e.g. moulded) as a single component with the first and second actuator gears. In this variant of the second and third embodiments, the actuating lever (instead of the outer cover) is connected to the drive coupling, so that rotation of the actuating lever causes rotation of the drive coupling. The outer cover and actuator may also include cooperating means configured so that, after inhalation, when the user rotates the outer cover back into its closed position in which it covers the mouthpiece, the actuator is also rotated back into its initial position. In other words, the outer cover is passive during opening, but is linked to the actuator during closing.

Instead of the indexing mechanism being disengaged before piercing, the indexing mechanism could be configured so that the indexing mechanism is disengaged after the blister piercer has begun to pierce the lid of a blister so that each piercing element is drawn across and through the lid of the blister as it enters it. This creates larger openings than those created when the strip is stationary during piercing, which can help to ensure that the drug dose is entrained in the airflow and removed from the blister.

Other opening means may be used instead of a piercer; for example the blisters may be opened by peeling the lid from the base, or by folding the blister so that the lid is burst open, as described in WO 16 / 083102.

In addition to the airflow through the blisters, the inhaler may also have one or more bypass airflow channels. Air flows through the bypass channel from outside the device and into the mouthpiece, without passing through the blister. The bypass airflow reduces the resistance of the device. The size of the bypass is chosen so that sufficient air nonetheless flows through the blisters to ensure complete evacuation of the powder.

Preferably the inhaler retains the used blisters. More preferably the inhaler has a wall to separate the interior of the housing into used and unused blister compartments. The wall is preferably rigid and slideably mounted so that the sizes of the unused and used blister compartments change relative to each other as the number of blisters that are used increases and the number of unused blisters decreases.

The used blisters are preferably crushed so that they take up less space. Thus the blister strip indexing wheel is preferably positioned such that the distance between the hub and the inner surface of the housing is less than the depth of a blister so that onward rotation of the wheel after piercing causes a blister to be at least partially squashed between the hub and the wall.

The inhaler may be either passive or active. In a passive inhaler, the dose is entrained in a flow of air caused when the user inhales through the mouthpiece. An active inhaler includes means for generating a pressurized flow of gas or air through the blister to entrain the dose and carry it out of the blister through the mouthpiece and into the user's airway. Although the term "mouthpiece" is used, the invention is also applicable to devices in which the dose is inhaled through the nasal passages.

## Claims

1. An inhaler comprising:
• a housing which contains a blister strip having a plurality of blisters which contain powdered medicament for inhalation,
• a mouthpiece mounted to the housing through which the medicament is inhaled by a user,
• an indexing and opening mechanism for indexing the blister strip and for opening the blisters which is operated by an actuator,
• wherein the blister strip is indexed by forward motion of the actuator from a first position to a second position,
**characterized in that** the blister strip is also indexed in the same direction by reverse motion of the actuator from the second positon to the first position.

2. An inhaler according to claim 1 wherein the indexing and opening mechanism is arranged to index the blister strip forwards by one blister on each of the forward and reverse motions of the actuator and to open two blisters which preferably contain different medicaments.

3. An inhaler according to claim 1 or claim 2, wherein the inhaler has an outer cover which is pivotally mounted on the housing.

4. An inhaler according to claim 3, wherein the outer cover forms the actuator, wherein in the first position the outer cover is closed so that the mouthpiece is covered, and in the second position the outer cover is open so that the mouthpiece is exposed, and so that motion of the outer cover causes indexing of the blister strip and opening of the blisters.

5. An inhaler according to any of claims 1 to 3, wherein the inhaler has a lever which forms the actuator so that motion of the lever causes indexing of the blister strip and opening of the blisters.

6. An inhaler according to any of claims 1 to 5 wherein the opening means comprises a piercer and the actuating mechanism indexes one or more blisters into alignment with the piercer for piercing.

7. An inhaler according to claim 6 wherein the blister strip is indexed during a first part of the forward motion of the actuator, the piercer pierces one or more aligned blisters during a second part of the forward motion, the piercer is removed during a first part of the reverse motion of the actuator and the blister strip is indexed again during a second part of the reverse motion.

8. An inhaler according to any of claims 1 to 7 wherein the indexing and opening mechanism comprises first and second drive gears, an idler gear and a blister strip indexing wheel, wherein the actuator is drivingly linked to the first drive gear, and to the second drive gear via the idler gear so that the first and second drive gears rotate in opposite senses during motion of the actuator; wherein the first drive gear drives the indexing wheel during at least part of the forward motion of the actuator, and does not drive the indexing wheel during the reverse motion of the actuator; and the second drive gear drives the indexing wheel during at least part of the reverse motion of the actuator, and does not drive the indexing wheel during the forward motion of the actuator.

9. An inhaler according to claim 8 wherein the indexing and opening mechanism comprises first and second actuator gears which are connected to and driven by the actuator, wherein the first actuator gear drives the first drive gear, and the second actuator gear drives the idler gear which in turn drives the second drive gear.

10. An inhaler according to claim 8 or claim 9 wherein the first and second drive gears are axially linked together to form a shuttle whilst being free to rotate independently, wherein the first and / or second drive gear has a track follower which interacts with a track formation on the housing to cause the shuttle to translate axially relative to the indexing wheel so that the first and second drive gears engage and disengage with the indexing wheel.

11. An inhaler according to claim 10, wherein the first and second drive gears each have a ramp follower which interacts with ramps on the housing to cause the first and second drive gears to disengage with the indexing wheel when the actuator is in the first or second position.

12. An inhaler according to claim 8 or claim 9 wherein a first drive coupling is connected to the first drive gear, a second drive coupling is connected to the second drive gear, and the drive couplings engage and disengage with the indexing wheel.

13. An inhaler according to any of claims 1 to 7 wherein the indexing and opening mechanism comprises first and second drive gears that are connected to a blister strip indexing wheel, and an idler gear, wherein the actuator is drivingly linked to the first drive gear during at least part of the forward motion of the actuator, and is not drivingly linked to the first drive gear during the reverse motion of the actuator; and the actuator is drivingly linked to the idler gear and the second drive gear during at least part of the reverse motion of the actuator, and is not drivingly linked to the second drive gear during the reverse motion of the actuator.

14. An inhaler according to claim 13 wherein a dual drive coupling is connected to and driven by the actuator, and the dual drive coupling goes into and out of driving linkage with the first and second drive gears.

15. An inhaler according to claim 14 wherein the indexing and opening mechanism comprises a first actuator gear which drives the first drive gear and a second actuator gear which drives the idler gear which in turn drives the second drive gear, so that the first and second actuator gears rotate in opposite senses during motion of the actuator, and wherein the dual drive coupling engages and disengages with the first and second actuator gears

16. An inhaler comprising a housing which contains a blister strip having a plurality of blisters which contain powdered medicament for inhalation, a mouthpiece mounted to the housing through which the medicament is inhaled by a user, an outer cover which is mounted on the housing so that it pivots between a first, closed position in which the outer cover covers the mouthpiece, and a second, open position in which the mouthpiece is exposed, and an indexing and piercing mechanism; wherein pivoting the outer cover from the closed position to the open position causes the blister strip to be indexed by one blister and causes two blisters to be pierced, and wherein pivoting the outer cover from the open position to the closed position also causes the blister strip to be indexed by one blister in the same direction.
